# EUROPEAN PATENT APPLICATION

(11) **EP 2 329 833 A1**
(43) Date of publication of application: **08.06.2011**
(21) Application number: 10013068.1
(22) Date of filing: 27.01.2005
(51) Int. Cl.: A61K 35/36, C12N 5/074

(54) **Methods of making and using skin-derived stem cells**

(30) Priority: 27.01.2004 US 539556 P
(62) Divisional of application: 05706434.7
(71) Applicant: The Hospital for Sick Children Research Institute, Toronto, Ontario M5G 1X8 (CA)
(72) Inventor: The designation of the inventor has not yet been filed
(74) Representative: Lahrtz, Fritz

(57) **Abstract**

The present invention describes a method for isolating skin derived precursor stem cells from hair follicles or dermal papillae and also describes the use of these cells for inducing hair growth.

## Description

### Field of the Invention

In general, the present invention relates to the field of stem cell biology.

### Background of the Invention

While adult mammalian stem cells were previously thought only to differentiate into cells of their tissue of origin, a number of recent reports have identified cultured adult stem cells that show a surprisingly diverse differentiation repertoire. Although at least some reports of multipotency are due to unanticipated cellular fusion events that occurred in vivo, compelling evidence still exists for the multipotency of a number of cultured adult stem cell populations. Perhaps the most striking examples of this multipotency derive from blastocyst injection studies, where both MAPC cells isolated after long-term culture of bone marrow cells and neural stem cells from the CNS contributed to many different developing tissues. However, one caveat to these studies is that this multipotency was demonstrated only after these stem cell populations had been expanded for significant periods of time in culture, raising the question of whether these results truly reflect previously unsuspected endogenous multipotent adult precursors, or whether they are the consequence of culture-induced dedifferentiation and/or reprogramming.

We have previously identified one such multipotent precursor cell population from adult mammalian dermis. These cells, termed SKPs for Skin-derived Precursors, can be isolated and expanded from rodent and human skin, and differentiate into both neural and mesodermal progeny, including into cell types that are never found in skin, such as neurons.

### Summary of the Invention

We have previously described the isolation of multipotent stem cells from juvenile and adult rodent skin, which we have termed as SKPs for skin-derived precursors. These cells derive from the dermis, and clones of individual cells proliferate and differentiate in culture to produce neurons, glia, smooth muscle cells and adipocytes. These cells have previously been described in PCT Patent Publication Nos. WO/0153461 and WO/03010243, hereby incorporated by reference.

Here, we show that multipotent stem cells are also found in the dermal papilla of hair follicles. Based on our discovery, the present invention features methods for purifying multipotent stem cells from the dermal papilla of hair follicles. Briefly, hair follicles (or portions of hair follicles containing the follicular dermal papilla) are obtained from a mammal. If desired, hair follicles may be further dissociated into smaller pieces by any method including, for example, enzymatic digestion or mechanical disruption. For example, the hair follicle may be dissociated to obtain the follicular dermal papilla. Hair follicles or portions thereof are next cultured in conditions under which multipotent stem cells grow and proliferate non-adherently and non-multipotent stem cells die or adhere to the culture substrate. Under such conditions, multipotent stem cells typically grow as part of floating three-dimensional structures. To collect multipotent stem cells, the non-adherent cells are separated from adherent cells. If desired, these non-adherent cells which contain the multipotent stem cells are further cultured in the same conditions as described above and collected following their separation from adherent cells until at least 30%, 40%, 50%, 60%, 70%, 80%, 90%, 95%, 99%, or even 100% of cells are multipotent stem cells (or progeny thereof).

Desirably, the multipotent stem cells of the invention express at least one, two, three, or more than three of the following molecular markers: nestin, WNT-1, vimentin, versican, fibronectin, S100β, slug, snail, twist, Pax3, Sox9, Dermo-1, and SHOX2. Such markers may be detected by any standard method known in the art including, for example, Northern blot analysis, RT-PCR, western blot analysis, in situ hybridization, and immunohistochemical analysis. Such methods are described in detail, for example, in Ausubel et al., Current Protocols in Molecular Biology, John Wiley & Sons, New York, NY, 1998, hereby incorporated by reference. The multipotent stem cells of the invention may also express increased levels (at least one-fold, two-fold, three-fold, or more) of slug, snail, twist, and Pax3 relative to central nervous system neural stem cells. Desirably, the multipotent stem cells of the invention do not express measurable levels of at least one, two, three, or more than three of the following molecular markers: tyrosinase, c-kit, tryp-1, and DCT, which are markers of melanoblasts and melanocytes. The multipotent stem cells may also not express measurable levels of one or more of the following markers of Schwann cells: MBP, P0, p75NTR, and SOX10. According to this invention, the level of a marker in a cell is considered as being "not measurable" if the marker in the cell cannot be detected by a method that under appropriate conditions detects the expression of the corresponding marker in a control cell. For example, the multipotent stem cells of the invention is considered not to express measurable levels of p75NTR since using the same RT-PCR analysis, p75NTR expression is undetectable in the cells of the invention but detectable in CNS neural stem cells.

Under appropriate conditions, multipotent stem cells purified from the dermal papilla of hair follicles may differentiate into various non-neural cells (e.g., hair follicle cell, bone cell, smooth muscle cell, or adipocyte) and neural cells (a neuron, astrocyte, Schwann cell, or oligodendrocyte). Accordingly, the present invention provides methods for inducing hair growth by providing to a mammal a population of cells, in which at least 30% of cells are stem cells purified from the dermal papilla of hair follicles. Alternatively, multipotent stem cells may be purified from the dermal papilla of hair follicles and following their differentiation into hair follicle cells in vitro, these cells are provided to the mammal being treated. According to this invention, cells may be provided to a mammal using any standard method in the art, such as those described, for example, in Unger et al., Skin Therapy Lett. 8:5-7, 2003. Accordingly, hair growth may be induced anywhere on the skin of the mammal (such as the head, face, legs, or arms of a mammal), even in areas where hair follicles are not usually found. Desirably, hair growth is induced in areas in which hair was previously present but has been lost. Mammals being treated according to the present invention may have a condition characterized by loss or lack of hair, including for example, alopecia, male pattern baldness, female pattern baldness, accidental injury, damage to hair follicles, surgical trauma, burn wound, radiation or chemotherapy treatment site, incisional wound, and donor site wound from skin transplant and ulcer. Alternatively, the mammal being treated may simply have a desire to modify physical appearance. Desirably, hair is induced by at least 10%, 20%, 30%, 40%, 50%, 60%, 70%, 80%, 90%, 95%, or 100% relative to an untreated control.

In another aspect, the invention features a method of inducing hair growth in a mammal (e.g., a human) by providing to the mammal a population of cells, wherein at least 30% of the cells are multipotent stem cells or progeny thereof and are capable of producing hair follicle cells.

In still another aspect, the invention features a method of regenerating skin in a mammal (e.g., a human) by providing to the mammal a population of cells, wherein at least 30% of the cells are multipotent stem cells or progeny thereof and are capable of regenerating skin.

In either of the foregoing aspects, the population of cells may be produced using a method described herein, or by any other method known in the art (e.g., one described in PCT Patent Publication Nos. WO/0153461 and WO/03010243). Desirably, at least 80%, 90%, 95%, or even 99% of the cells are multipotent stem cells or progeny thereof and are capable of producing hair follicle cells or regenerating skin. In one embodiment, the stem cells are substantially purified from hair follicles or dermal papilla-containing portions thereof. By "substantially purified" is meant that the desired cells are enriched by at least 30%, more preferably by at least 50%, even more preferably by at least 75%, and most preferably by at least 90% or even 95%. Desirably, the multipotent stem cells express at least one protein selected from the group consisting of nestin, WNT-1, vimentin, fibronectin, S100β, slug, snail, twist, Pax3, Sox9, Dermo-1, and SHOX2. Also desirably, the multipotent stem cells do not express measurable levels of at least one protein selected from the group consisting of tyrosinase, c-kit, tryp1, DCT, MBP, P0, SOX10, and p75NTR.

In another aspect, the invention features a kit that includes multipotent stem cells capable of inducing hair growth in a mammal and instructions for inducing hair growth in a mammal.

In yet another aspect, the invention features a kit comprising multipotent stem cells capable of regenerating skin in a mammal and instructions for regenerating skin in a mammal.

In either of the foregoing aspects, the cells may be produced using a method described herein, or by any other method known in the art (e.g., one described in PCT Patent Publication Nos. WO/0153461 and WO/03010243). In one embodiment, the stem cells are substantially purified from hair follicles or dermal papilla-containing portions thereof. Desirably, the multipotent stem cells express at least one protein selected from the group consisting of nestin, WNT-1, vimentin, fibronectin, S100β, slug, snail, twist, Pax3, Sox9, Dermo-1, and SHOX2. Also desirably, the multipotent stem cells do not express measurable levels of at least one protein selected from the group consisting of tyrosinase, c-kit, tryp1, DCT, MBP, P0, SOX10, and p75NTR.

In another aspect, the invention features a method of making hair follicles. This method includes the steps of culturing multipotent stem cells under conditions that induce the stem cells to differentiate into hair follicles. The cells may be produced using a method described herein, or by any other method known in the art (e.g., one described in PCT Patent Publication Nos. WO/0153461 and WO/03010243). In one embodiment, the stem cells are substantially purified from hair follicles or dermal papilla-containing portions thereof. Desirably, the multipotent stem cells express at least one protein selected from the group consisting of nestin, WNT-1, vimentin, fibronectin, S100β, slug, snail, twist, Pax3, Sox9, Dermo-1, and SHOX2. Also desirably, the multipotent stem cells do not express measurable levels of at least one protein selected from the group consisting of tyrosinase, c-kit, tryp1, DCT, MBP, P0, SOX10, and p75NTR.

In all foregoing aspects of the invention, multipotent stem cells may be purified from the dermal papilla of hair follicles from any post-natal mammal, including an adult or juvenile mammal (e.g., human). Preferred mammals include, for example, humans, non-human primates, mice, pigs, and rats. If these cells are used in transplantation procedures, the donor mammal is desirably immunologically similar to the recipient mammal. Even more desirably, these cells are obtained from an autologous source. Optionally, the cell of the invention may contain a heterologous gene in an expressible genetic construct. Such a gene may encode, for example, a therapeutic protein, a protein which induces or facilitates differentiation of the stem cell, a growth factor, or an anti-apoptotic protein.

### Brief Description of the Figures.

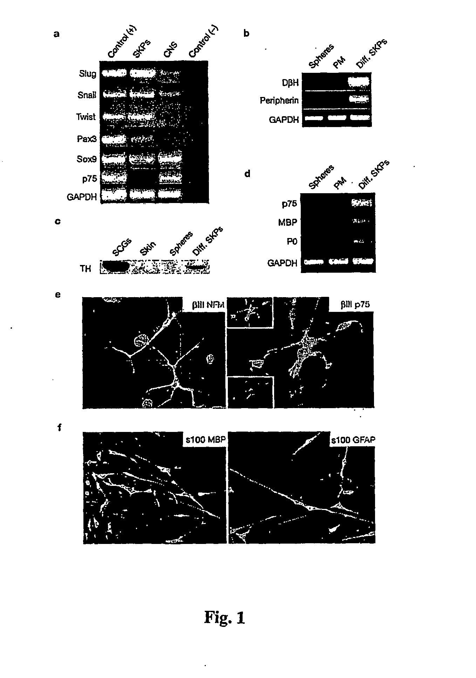

Figs. 1A-1F are photographs showing SKPs express markers of embryonic neural crest and differentiate into peripheral neurons and Schwann cells. Fig. 1A shows RT-PCR results for genes involved in embryonic neural crest determination and migration in total RNA isolated from SKP spheres compared to embryonic telencephalic neurospheres (CNS), both of which were cultured in the presence of FGF2 plus EGF. The positive control (+ve) was RNA from E12 neural tube. RT-PCR for GAPDH was used as a loading control, and the last lane (H₂O) was run as a negative control. Fig. 1B shows RT-PCR for two markers of peripheral catecholaminergic neurons, dopamine-β-hydroxylase (DβH) and peripherin, in murine SKPs differentiated for 1 week in 10% serum (Diff.). SKP spheres and dissociated SKPs plated in proliferation medium (Prolif.) do not express these mRNAs. Fig. 1C shows western blot analysis for tyrosine hydroxylase (TH) in murine SKP spheres versus SKPs differentiated for 14 days in 10% serum supplemented with neurotrophins. The positive control was protein isolated from cultured sympathetic neurons of the superior cervical ganglion (SCGs). Fig. 1D shows RT-PCR for three markers of peripheral Schwann cells, p75NTR, myelin basic protein (MBP) and P0 peripheral myelin protein (P0), in total RNA from undifferentiated and differentiated rat SKPs. Fig. 1E shows immunocytochemical analysis of differentiated murine SKPs for markers of peripheral neurons. The left panel shows morphologically complex differentiated cells that co-express the neuronal markers βIII-tubulin and NFM. The right panel shows differentiated cells that co-express βIII-tubulin and p75NTR, proteins expressed by virtually all peripheral neurons. Fig. 1F shows immunocytochemical analysis of differentiated SKPs, showing that a subset of bipolar cells co-express S100β and MBP (left panel), or S100β and GFAP (right panel).

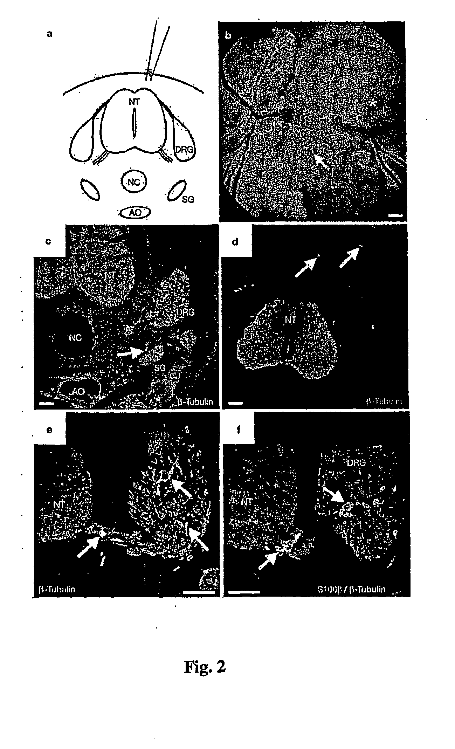

Figs. 2A-2F show SKPs migrate like neural crest cells when transplanted in ovo. Fig. 2A is a schematic illustration of a cross-section through a stage 30 chick embryo showing some of the major anatomical landmarks near, or associated with, the neural crest migratory stream. Fig. 2B is a photomicrograph of a single YFP-positive sphere (arrow) transplanted into a chick embryo in ovo. The asterisk indicates the head of the embryo. Fig. 2C-2F are cross-sections of stage 30 chick embryos, immunolabeled for βIII-tubulin, show that the transplanted SKPs had migrated to the sympathetic ganglia (c, arrow), skin (d, arrows), spinal nerve and DRG (e, arrows). Fig. 2F depicts immunohistochemical analysis for βIII-tubulin and S100β showing that many of the transplanted SKPs in both the spinal nerve and DRG (arrows) express S100β at stage 30. NT, neural tube; NC, notochord; DRG, dorsal root ganglia; SG, sympathetic ganglia; AO, aorta; g, gut. Calibration bars = 100 µm.

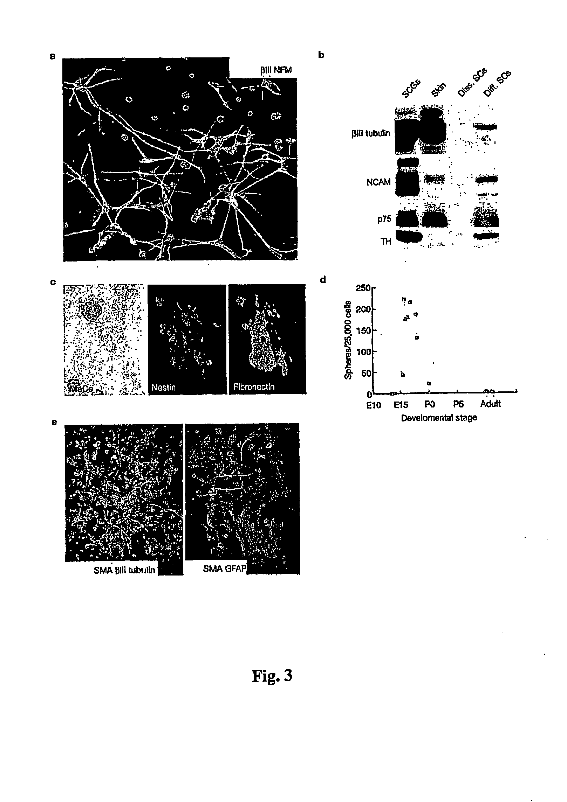

Figs. 3A-3E show multipotent endogenous SKPs are abundant in skin during late embryogenesis and persist into adulthood. Fig. 3A is a double-labelling of differentiated primary E18 murine skin cells with antibodies for βIII-tubulin and NFM. Note that more cells express βIII-tubulin than NFM, since these are early and late neuronal markers, respectively. Fig. 2B is a western blot analysis for proteins expressed in peripheral neurons in E18 skin cells differentiated under conditions similar to those in Fig. 3A. For comparison, equal amounts of protein were analysed from lysates of whole skin at the same age (skin), dissociated E18 skin cells that were not differentiated (Diss. SCs), and from primary cultures of peripheral sympathetic neurons (SCGs). Fig. 3C is a phase contrast micrograph of primary SKP spheres grown immobilized in methylcellulose (left panel). The two other panels are fluorescent photomicrographs of a primary sphere grown in methylcellulose that was double-labelled with antibodies to nestin and fibronectin. Fig. 3D is a graph depicting quantitation of the number of cells that give rise to primary SKP spheres in murine back skin isolated at various developmental ages ranging from embryonic day 13 to adulthood. Cell numbers are expressed relative to a given number of primary skin cells. Fig. 3E shows an immunocytochemical analysis of primary murine SKP clones, one for βIII-tubulin and smooth muscle actin (SMA), and one for SMA and the glial cell marker GFAP, demonstrating that these primary SKPs clones can generate both neural and mesodermal progeny.

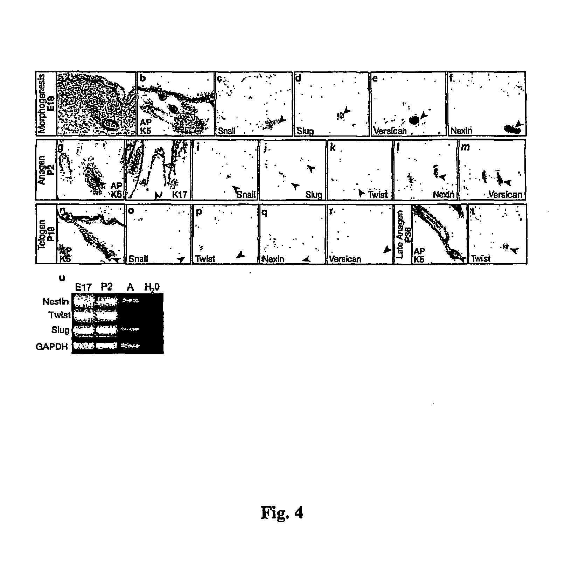

Figs. 4A-4U show expression of SKP transcription factors is localized to the follicle papillae of dorsal hair follicles and is hair-cycle dependent. Arrowheads indicate follicle papillae. At E18.5, a dorsal hair follicle in late morphogenesis has a well-developed dermal papilla ensheathed by epidermal progenitor (matrix) cells (Fig. 4A). These follicle papillae express high levels of endogenous alkaline phosphatase (AP) (Fig. 4B), as well as elevated versican (Fig. 4E) and nexin expression (Fig. 4F. Overlapping localized expression of SKP markers snail (Fig. 4C) and slug (Fig. 4D) is observed in follicle papillae. In P2 postnatal skin, hair follicles continue to mature as they approach the first synchronized anagen phase. Expression of SKP markers snail (Fig. 4I), slug (Fig. 4J) and twist (Fig. 4K) continues to localize to the follicle papillae, marked with high levels of alkaline phosphatase (Fig. 4G), nexin (Fig. 4L) and versican (Fig. 4M) expression, but do not overlap with Keratin 17 (K17; Fig. 4H), a marker for the outer root sheath (arrowhead) and the bulge epidermal stem cell niche or with Keratin 5 (K5; Fig. 4G), a basal epidermal layer marker. At P19, hair follicles are in the first synchronized telogen phase and associated follicle papillae are small structures at the distal tip of resting follicles. While these structures express low levels of alkaline phosphatase (Fig. 4N), telogen follicle papillae do not express the anagen markers nexin (Fig. 4Q) or versican (Fig. 4R), or the SKP markers snail (Fig. 4O) or slug, although very low levels of twist (Fig. 4P) are detected. As shown in serial sections of P36 skin, in the next wave of anagen, SKP markers are re-expressed with anagen markers in the follicle papillae of growing follicles (Figs. 4S, 4T). Fig. 4U shows SKP markers nestin, twist, and slug in developing (late gestation E17 and early postnatal P2) and adult skin are detected by RT-PCR.

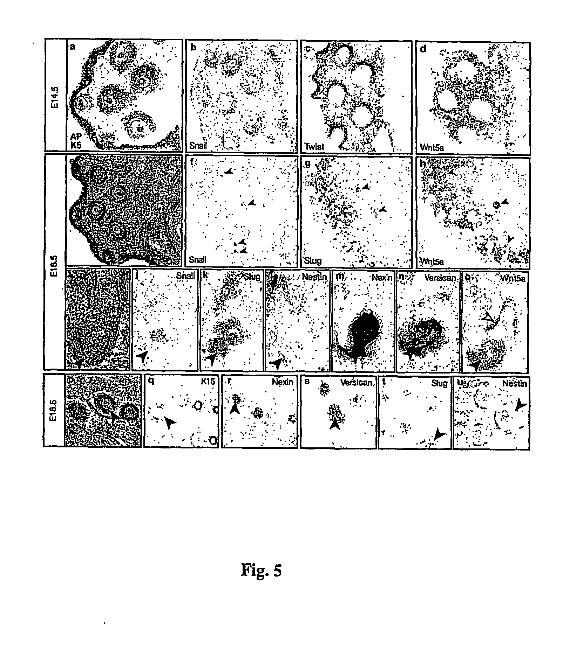

Figs. 5A-5U show neural crest-derived facial dermis dynamically expresses SKP markers, which become progressively restricted to follicle papillae of vibrissae and hair follicles. Arrowheads indicate follicle papillae. Figs. 5A-5D are serial sections of E14.5 whisker pad. Concentric rings characteristic of cross-sectioned developing vibrissae follicles illustrate epidermal downgrowths (Keratin5; K5) and associated dermal condensates (alkaline phosphatase; AP) (Fig. 5A). These dermal condensates express higher levels of SKP markers snail (Fig. 5B), slug, and twist (Fig. 5C) than surrounding dermis, as well as anagen papilla markers nexin and versican. Wnt5a expression, another marker of papilla development, is expressed throughout the vibrissae pad dermis (Fig. 5D). Fig. 5E-5O show vibrissae pad cross-section at low magnification (Figs. 5E-5H, serial sections) and high magnification photomicrographs of adjacent sections through a single vibrissae follicle longitudinally sectioned from an E16.5 embryo (Figs. 5I-5O, serial sections). Interspersed with deeply-penetrating concentric rings of vibrissae follicles which have engulfed associated follicle papillae, smaller hair follicles are observed (arrowheads; Fig. 5E). Localized expression of SKP markers snail (Figs. 5F, 5J), slug (Figs. 5G, 5K), nestin (Fig. 5L), and twist is detected in papillae of vibrissae and hair follicles, along with high levels of papilla markers nexin (Fig. 5M) and versican (Fig. 5N). In addition to follicle papilla expression (Fig. 5H, 5O), Wnt5a is also diffusely detected in the upper dermis and in the outer root and inner root sheath (arrowhead, Fig. 5O) of vibrissae. Figs. 5P-5U are serial sections of E18.5 whisker pad, where Figs. 5P-5S are serial sections, and Figs. 5T-5U are serial sections. By E18.5, vibrissae papillae (Fig. 5P) continue to express high levels of nexin (Fig. 5R) and versican (Fig. 5S), but reduced expression of SKP markers, slug (Fig. 5T) and nestin (Fig. 5U). The epidermal stem cell marker Keratin15 is expressed strongly in the outer root sheath and at low levels in the matrix adjacent to the papillae, but not in the papillae.

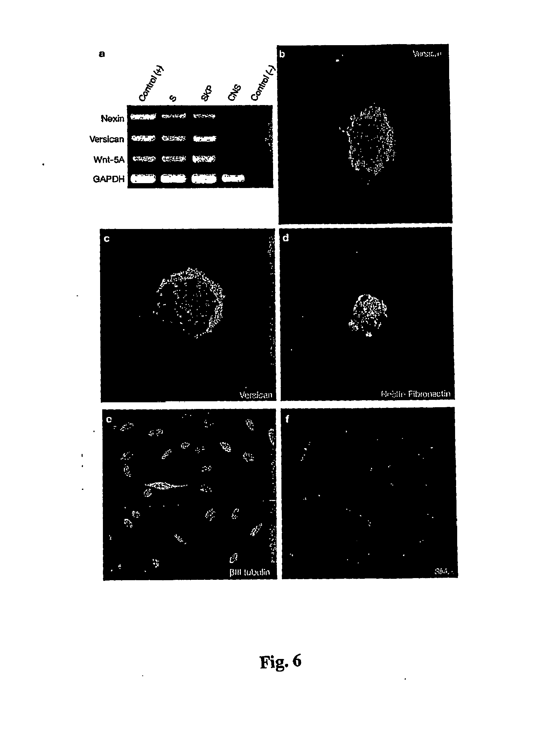

Figs. 6A-6F show adult whisker papillae contain SKP-like cells. Fig. 6A shows RT-PCR for the follicle papilla markers nexin, versican and Wnt-5a in total RNA isolated from E12 embryos (+ve), skin (S), SKP spheres (SKP) or CNS neurospheres (CNS). SKPs but not neurospheres express all of these markers. Fig. 6B shows immunocytochemical analysis for the follicle papilla marker versican in a SKP sphere. Most, if not all of the cells express versican. Figs. 6C and 6D show immunostaining of whisker papilla spheres for versican (Fig. 6C), or for nestin and fibronectin (Fig. 6D). Figs. 6E and 6F are fluorescence photomicrographs of differentiated whisker papilla spheres differentiated for 3 days and then immunostained for βIII-tubulin (Fig. 6E) or SMA (Fig. 6F).

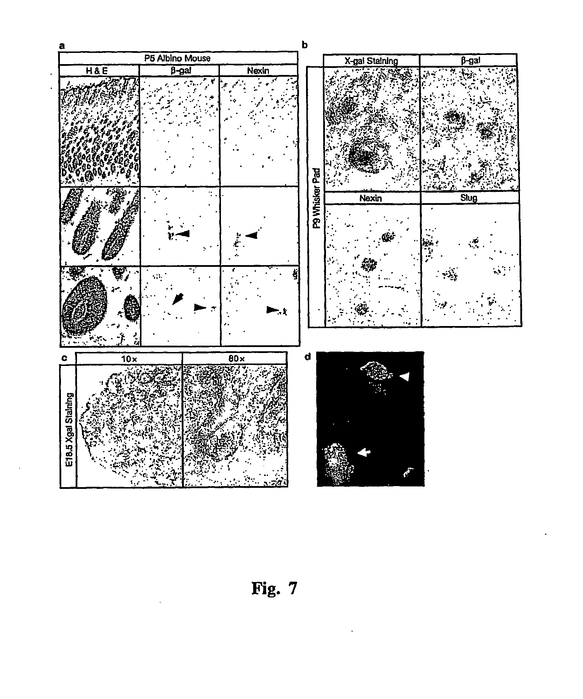

Fig. 7A is a series of photomicrographs showing overlapping expression of β-galactosidase and the dermal papilla marker nexin, in postnatal day 5 (P5) neonatal Wnt1Cre;Rosa26R dorsal skin of an albino mouse. Faint β-galactosidase-positive cells observed in the lower matrix (arrow) are likely melanoblasts, wherease strong β-galactosidase-positive cells that co-express nexin in the adjacent serial sections are found in the follicle papillae (arrowheads), and potentially represent NCSC-derived cells that are the in vivo source of SKPs. In all cases, the left, middle and right panels are photomicrographs of adjacent serial sections, with the left panel being hematoxylin and eosin-stained (H & E), and the middle and right panels being hybridized with probes specific to β-galactosidase and nexin mRNAs, respectively.
Figs. 7B-7D are photographs showing whisker papillae are of neural crest origin. Fig. 7B shows sections through the whisker pad of a P9 Wnt1Cre;R26R mouse showing cross-sections of vibrissae at the level of the papillae that were either stained for β-galactosidase activity using X-gal, or that were analyzed for expression of β-galactosidase, nexin and slug mRNAs by in situ hybridization. All of these genes were expressed in the whisker papillae, and many cells outside of the follicle in the dermis were also β-galactosidase positive, consistent with the neural crest origin of facial dermis. Fig. 7C shows sections of the whisker pad from an E18.5 Wnt1Cre;R26 mouse that were X-gal stained, and photomicrographs taken at different magnifications. At both developmental stages, the whisker papillae are completely labelled, indicating the most of the cells in these papillae are neural crest-derived. Fig. 7D shows dissected papillae from a Wnt1Cre;R26R mouse (arrow) and from its wild-type littermate (arrowhead) that were stained with X-gal to detect β-galactosidase activity.

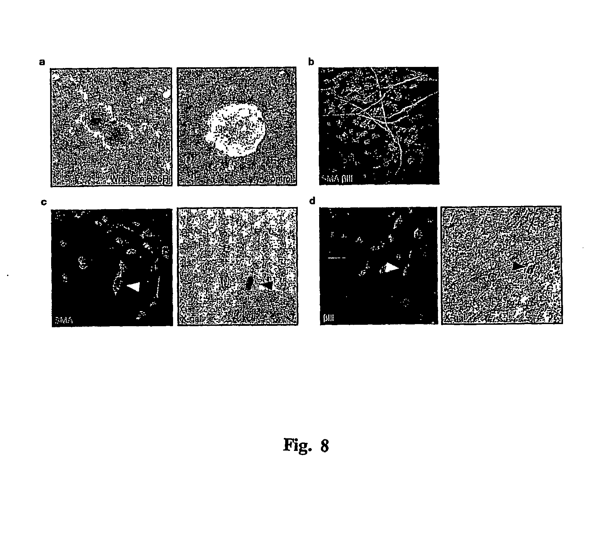

Figs. 8A-8D show SKPs derived from facial skin are of neural crest origin. Fig. 8A shows X-gal stained SKP spheres generated from the whisker pad skin of a litter of neonatal Wnt1Cre;R26 mice (left panel) or, as a control, from dorsal skin of a neonatal rat. Since the transgene is not penetrant in facial skin of all Wnt1Cre;R26 mice, whisker pads were initially stained with X-gal to assay transgene expression, and then SKPs were generated from the contralateral whisker pad of animals where penetrance was high. All of the sphere cells were transgene-positive. The spheres depicted were passaged two times. Fig. 8B shows double-label immunocytochemistry for SMA and βIII-tubulin on a single isolated Wnt1Cre;R26R-positive whisker pad SKP sphere differentiated for two weeks. Like dorsal skin SKPs, both SMA-positive smooth muscle cells and βIII-tubulin-positive neurons were generated. Figs. 8C and 8D are photomicrographs of transgene-positive isolated Wnt1Cre;R26R whisker pad spheres that were differentiated for two weeks, stained for X-ga1, and then immunostained for either SMA or βIII-tubulin. In each pair, the left panel is the fluorescence photomicrograph, and the right a brightfield photomicrograph showing the same field. Arrows denote the same cell in both panels. While the β-galactosidase transgene was downregulated in most cells during differentiation (all of the parent sphere cells were transgene-positive), some smooth muscle cells and neurons still expressed the nuclear transgene.
Fig. 9A show immunocytochemical analysis of SKP spheres cultured from neonatal mouse (nestin, fibronectin, Sca-1) or rat (p75NTR) back skin, and passaged two or three times (top panels) as compared to neurospheres cultured from the embryonic telencephalon and passaged a similar number of times (bottom panels). SKPs express fibronectin and Sca-1 but not p75NTR, whereas neurospheres do not express fibronectin or Sca-1, but do express p75NTR.
Fig. 9B shows RT-PCR analysis for Dermo-1 and SHOX2, transcription factors involved in dermal and craniofacial development, in RNA from SKPs and CNS neurospheres as described in Fig. 1. The positive control (+ve) was RNA from E16 forelimb.
Fig. 9C shows a western blot analysis for NCAM and dopamine-β-hydroxylase in murine SKP spheres versus SKPs differentiated for 14 days in 10% serum supplemented with neurotrophins. The positive control was protein isolated from cultured peripheral sympathetic neurons from the superior cervical ganglion (SCGs).
Fig. 9D shows immunostaining of differentiated SKPs shows that a subset of differentiated cells express tyrosine hydroxylase (TH).
Fig. 10A shows immunocytochemical analysis for βIII-tubulin in primary E18 (middle panel) or adult (right panel) murine skin cells versus E18 murine CNS telencephalic cells (left panel). All cells were plated in FGF2 and 10% serum for 7 days and differentiated in medium containing 5% serum supplemented with neurotrophins.
Fig. 10B shows a series of phase contrast micrographs of primary murine SKP spheres grown for 1 week at varying concentrations of starting cells (c/ml).
Fig. 10C is a graph showing quantitation of the number of cells that give rise to primary SKP spheres in murine back skin isolated at various developmental ages ranging from embryonic day 13 to adulthood. Cell numbers were normalized to the total number of cells present in a back skin isolation.
Fig. 10D shows immunocytochemical analysis for nestin in a primary murine SKP clone plated onto poly-d-lysine/laminin.
Fig. 10E shows immunocytochemical analysis for βIII-tubulin in a primary murine SKPs clone grown in methylcellulose and then differentiated.
Fig. 10F shows a series of micrographs of primary SKP spheres generated by mixing very small numbers of YFP-positive adult vibrissal cells and E18 primary skin cells and culturing in the presence of FGF2 and EGF for one week. The left panel is a phase micrograph and the right panel a fluorescence micrograph of the same field. The arrowhead indicates a YFP-positive sphere.
Fig. 10G shows immunocytochemical analysis of primary SKP spheres for markers of melanoblasts and hematopoietic stem cells. The two left panels were immunostained for nestin (arrows) and for trp1 (arrowheads), with the top panel being total dissociated skin cells, and the bottom a primary SKP sphere. The two right panels were immunostained for c-kit, with the top panel being a cytospin of a bone marrow aspirate (positive cells are marked with arrowheads), and the bottom panel a primary SKPs sphere.
Fig. 10H shows a series of fluorescence micrographs of primary dissociated dorsal skin cells double-labelled for the precursor cell marker Sca-1 and the Schwann cell markers GFAP, myelin basic protein (MBP), and CNPase, or the melanoblast/melanocyte markers c-kit, trp1 and dct.
Fig. 11A shows a series of photomicrographs of a vibrissal papilla dissection from an adult pigmented mouse. The left picture shows the intact vibrissa, while the other pictures show the dissection. The arrowhead indicates the papilla.
Figs. 11B and 11C show immunocytochemical analysis of dissected whisker papillae for Sca-1 and dct.
Figs. 11D and 11E show fluorescence photomicrographs of dissociated papilla cells that were immunostained for keratin 18 (Fig. 11D) or βIII-tubulin and CNPase (Fig. 11E).
Fig. 11F shows immunocytochemical analysis for nestin and pni-tubulin in adult vibrissal papilla cells directly differentiated for 7 days in FGF2, 10% serum and neurotrophins.
Fig. 11G shows a series of phase photomicrographs of dissociated papilla cells that were cultured for one week in the presence of FGF2 plus serum or chick embryo extract (CEE). In the presence of CEE, floating spheres of cells were generated.
Fig. 11H shows a fluorescence photomicrograph of a whisker papilla sphere that was differentiated on an adherent substratum for 3 days and then immunostained for βIII-tubulin.

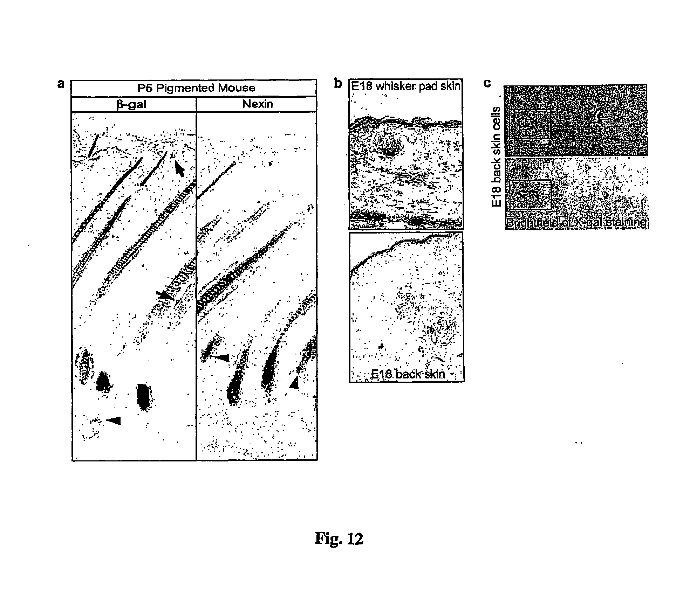

Fig. 12A shows overlapping expression of β-galactosidase and the dermal papilla marker, nexin, in postnatal day 5 (P5) Wnt1Cre;Rosa26 dorsal skin from pigmented mice. Pigmented melanin granules are deposited in the developing hair shaft by neural crest-derived melanoblasts resident and interspersed in the matrix. A subpopulation of follicle papilla cells express mRNAs for both β-galactosidase (arrowhead) and nexin (arrowhead). Beyond the outer root sheath, occasional β-galactosidase-positive cells can also be seen in the dermal sheath (lower arrow), a structure closely associated with follicle papillae. Single β-galactosidase-positive cells are seen emerging from the upper portions of the follicle into the interfollicle epidermis and are likely melanoblasts (upper arrow).
Fig. 12B shows expression of the β-galactosidase transgene in E18 whisker pad versus E18 back skin from Wnt1Cre;Rosa26R embryos, as detected by X-gal staining of cryostat sections. Similar results were obtained at a variety of developmental ages using both X-gal staining and in situ hybridization for the β-galactosidase transgene. Few transgene-positive cells were detected in dorsal skin, in spite of the fact that there are abundant melanoblasts and Schwann cells at this point.
Fig. 12C shows photomicrographs of dissociated back skin cells from neonatal Wnt1Cre;R26R mice that were stained with X-gal one day after plating. The upper panel is a phase micrograph while the lower is a brightfield micrograph of the same field showing that neural crest-derived pigmented melanocytes (boxed) were β-galactosidase negative.
Figs 13A and 13B are photomicrographs showing that differentiation of SKPs in media supplemented with serum and forskolin leads to the generation of parallel arrays of S100β positive cells, characteristic of Schwann cells (Fig. 13B), while few S100β positive cells are found in cultures differentiated in serum alone (Fig. 13A).
Figs. 13C and 13D are photomicrographs showing that SKP-derived Schwann cells coexpress myelin proteins MBP (Fig. 13C) and pmp22 (13D).
Fig. 13E is a photomicrograph showing that SKP-derived Schwann cells could be readily observed under phase microscopy after being differentiated in serum-free media supplemented with N2, heregulin-β and forksolin.
Fig. 13F is a photomicrograph showing that colonies of SKP-derived Schwann cells could be isolated with cloning cylinders and replated in new dishes.
Fig. 13G is a photomicrograph showing that isolated and replated Schwann cells could be expanded to yield cultures of greater than 95% purity.
Fig. 13H is a photomicrograph showing that single, clonal SKPs spheres can be differentiated in serum-free media supplemented with N2, heregulin-β and forksolin to produce cultures containing GFAP-positive Schwann cells (Fig. 13H).
Figs. 14A and 14B are photomicrographs showing that YFP-tagged SKP-SCs associate with neuronal processes.
Figs. 14C and 14D are photomicrographs showing SKP-SCs populate neuronal processes.
Fig. 14E is a photomicrograph showing that pmp22-positive myelinated fibers comprised of YFP-positive SKP-SCs were observed following co-culturing with DRG neurons with the addition of ascorbic acid and IGF-1 to the media, factors that induce myelination in vitro.
Fig. 14F is a photomicrograph showing that SKP-SCs associated with neuronal processes expressed Ki67.
Fig. 15A is a photomicrograph of a cerebellar organotypic slice culture from shiverer mice.
Figs. 15B and 15C are photomicrographs showing MBP expression in wild-type (Fig. 15B) and shiverer (Fig. 15C) mice.
Fig. 15D is a photomicrograph showing that transplanted cells cultured from wild-type animals can be identified in shiverer mice by the expression of MBP by the transplanted cells.
Figs. 15E and 15G are photomicrographs showing that transplanted SKP-SCs cultured from YFP-transgenic mice express MBP.
Fig. 15F is a photomicrograph showing that SKP-SCs transplanted into shiverer slices co-express s100β and MBP.
Figs. 16A and 16B are photomicrographs of transverse sections stained with myelin protein antibodies. Confocal microscopy revealed that the SKP-SCs expressed MBP (Fig. 16A) and pmp22 (Fig. 16B) with a morphology consistent with the profile of myelinated axons.
Fig. 16C is a series of adjacent optical slices showing pYFP-tagged transplanted cells engulfing neurofilament positive axons.
Fig. 16D is a series of photomicrographs showing that after 1-2 weeks, transplanted cells had migrated proximally and distally from the site of transplantation and were aligned longitudinally in the nerve.
Fig. 17 shows an image from a mouse-brain atlas. The box indicates the inferior colliculus.
Fig. 18 depicts semithin sections (1 µm) stained with toluidine blue. At 100x magnification on a light microscope darkly stained myelin profiles can be observed (arrow heads) in the transplanted brain (right panel) but not the non-transplanted shiverer brain control (left panel).
Fig. 19 shows examples of normal myelin (left panel) and shiverer myelin (right panel).
Fig. 20 is an electron micrograph demonstrating compact myelin wrapping host axons (arrow heads) of in the transplanted brain (right panel) but not the non-transplanted shiverer brain control (left panel).
Fig. 21 is an electron micrograph demonstrating compact myelin wrapping host axons (arrow heads) of in the transplanted brain (right panel) but not the non-transplanted shiverer brain control (left panel). The inset in Fig. 21 shows the repeating major dense lines characteristic of compact myelin.
Fig. 22 demonstrates that SKPs integrate into the epidermis and dermis, and into specialized structures such as the follicular dermal papillae and dermal sheath.
Fig. 23 shows that SKPs express markers of follicular papillae.
   Colocalization of SKPs in follicular papillae with versican, a chondroitin sulfate proteoglycan highly expressed within the cells of the papillae, particularly during anagen phase of the hair cycle.
Fig. 24 is a high magnification confocal laser scanning microscopy image demonstrating that SKPs express versican.
Fig. 25 shows transplanted SKPs express PECAM, a marker of endothelial cells lining blood vessels.
Fig. 26 demonstrates that SKPs express markers of Schwann cells following transplant into the skin. A SKP-derived cell found in the dermis, expresses pmp22, a marker of peripheral Schwann cells.
Fig. 27 shows SKPs are found in the dermal sheath surrounding the base of the hair follicle, and express low levels of p75.
Fig. 28 shows that the dermal sheath is comprised of specialized fibroblasts and forms a collagen rich matrix. SKPs found within the dermal sheath also express collagen type I, which is expressed by endogenous dermal sheath cells.

### Detailed Description

A key question in the stem cell field is whether cultured multipotent adult stem cells represent bona fide endogenous multipotent precursor cells. Here we address this question, focussing on SKPs, a cultured adult stem cell from the dermis that generates both neural and mesodermal progeny. We show that SKPs derive from endogenous adult dermal precursors that share attributes with embryonic neural crest stem cells. We demonstrate that these endogenous SKPs first arise in skin during embryogenesis and persist in lower numbers into adulthood, with a niche in hair follicles. Furthermore, in transgenic mice that express β-galactosidase in neural crest progeny, tagged cells were detected within hair follicles, including within the dermal papillae. SKPs are multipotent stem cells that colonize peripheral tissues such as skin, during embryogenesis and maintain multipotency into adulthood. In addition, there are several populations of stem cells in the skin, including keratinocyte stem cells and follicle bulge stem cells. There may be a form of communication of cells between the bulge and dermal papilla during the hair growth cycle.

### Example 1: SKPs share characteristics with and have potential similar to embryonic neural crest stem cells

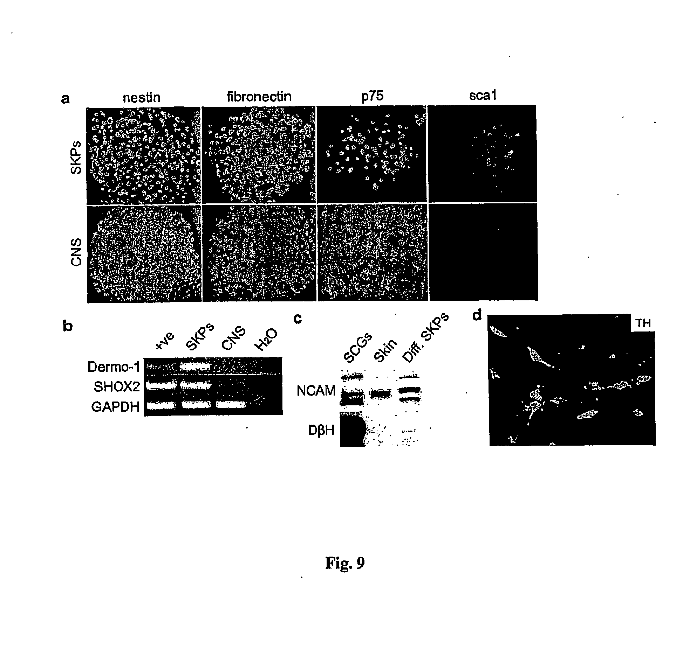

To characterize the origin of SKPs, we first compared them to stem cell populations that generate neural and/or mesodermal progeny. Since we previously demonstrated that SKPs are distinct from mesenchymal stem cells, we focused upon CNS neural stem cells and embryonic NCSCs. Immunocytochemical comparison of SKPs and embryonic CNS neurospheres revealed that the two populations were distinct. Both expressed nestin and vimentin, but only SKPs expressed fibronectin and the precursor cell marker Sca-1, while only neurospheres contained cells expressing p75NTR (Fig. 9A). We then analysed SKPs for expression of genes associated with embryonic NCSCs. RT-PCR analysis revealed that SKPs expressed the transcription factors slug, snail, twist, Pax3, and Sox9, genes expressed in various populations of embryonic NCSCs in vivo (Fig. 1A). Except for Sox9, all these genes were expressed at lower or undetectable levels in embryonic CNS neurospheres (Fig. 1A). SKPs also expressed the transcription factors Dermo-1 and SHOX2, which are expressed in embryonic dermis and craniofacial regions (Fig. 9B). A similar pattern of gene expression was observed in embryonic, neonatal and adult SKPs passaged from 1 to 15 times. Thus, SKPs express genes characteristic of embryonic NCSCs and/or their embryonic derivatives.

We next tested whether SKPs differentiated into cell types that are exclusively neural crest-derived during embryogenesis, such as peripheral catecholaminergic neurons and Schwann cells. For catecholaminergic neurons, SKPs were differentiated one to three weeks under conditions used to differentiate embryonic NCSCs into peripheral neurons. Immunocytochemistry, RT-PCR and western blots revealed a subpopulation of differentiated cells with neuronal morphology that co-expressed the pan-neuronal markers βIII-tubulin and neurofilament M (NFM) (Fig. 1E), and proteins typical of peripheral neurons, including p75NTR (Fig. 1E), peripherin (Fig. 1B), NCAM (Fig. 9C) and the catecholaminergic markers tyrosine hydroxylase and dopamine-β-hydroxylase (Figs. 1B, 1C, 9C, 9D). These neurons were generated by differentiated embryonic and neonatal SKPs, and by SKPs passaged from 1 to 20 times.

We previously reported that SKPs differentiate into bipolar cells co-expressing GFAP and CNPase, consistent with a Schwann cell phenotype. Further characterization demonstrated that these cells also expressed S100β and p75NTR (Figs. 1D, 1F), and, when differentiated in forskolin to elevate intracellular cAMP, myelin basic protein (MBP) and P0 (Figs. 1D, 1F), as reported for cultured Schwann cells.

To ask whether SKPs exhibited neural crest potential in vivo as well as in vitro, we generated SKPs from back skin of neonatal actin:YFP mice, and after 0 or 1 passage, transplanted single spheres of 200-250 cells into the chick neural crest migratory stream in ovo at Hamburger and Hamilton stage 18 (Figs. 2A, 2B). Analysis three days later (H&H stage 30) revealed that approximately half the transplanted cells migrated into peripheral neural crest targets, while very few migrated into the neural tube. Many YFP-positive cells were present in the spinal nerve and dorsal root ganglia, and some migrated to peripheral nerves or to the vicinity of the sympathetic ganglia (Figs. 2C, 2E). Interestingly, some YFP-positive cells were detected in the dermal layer of the skin (Fig. 2D). Of the SKPs in the DRG and spinal nerve, a subpopulation expressed S100β, a marker for Schwann cells (Fig. 2F). Thus, transplanted SKPs migrated along neural crest migratory pathways into neural crest-derived structures.

### Example 2: SKPs arise during embryogenesis and are maintained into adulthood

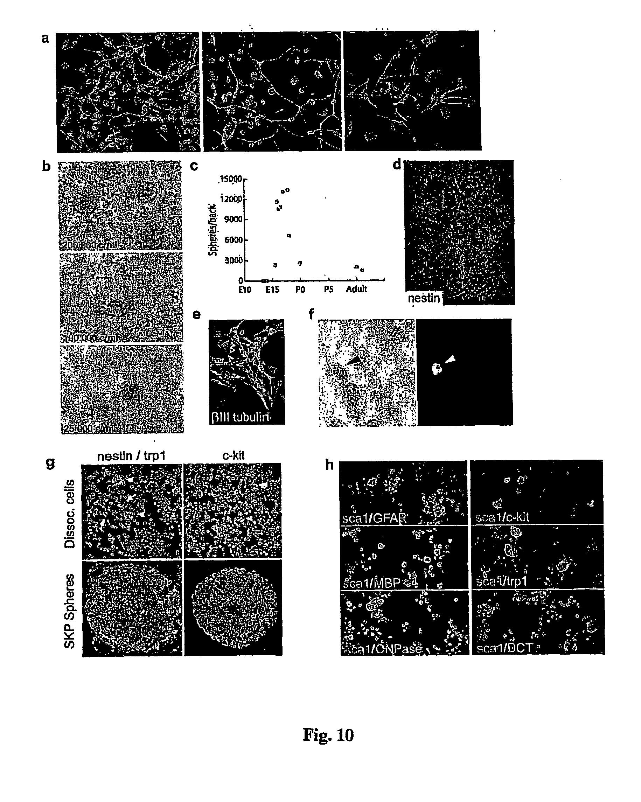

The foregoing data suggested that SKPs are embryonic neural crest-related precursors that arise in the dermis during embryogenesis and persist into adulthood. To test this hypothesis, we asked whether skin contained an endogenous precursor cell that could differentiate into neurons, a cell type never found in skin. Skin cells from embryonic (E18) or adult mice were dissociated and immediately differentiated under conditions that promote neuronal differentiation from embryonic NCSCs. Immunocytochemistry (Fig. 3A, Fig. 10A) and western blot analysis (Fig. 3B) revealed that some differentiated primary skin cells exhibited neuronal morphology and co-expressed βIII-tubulin, NFM, and p75NTR, a phenotype comparable to SKPs-derived peripheral neurons. Moreover, these differentiated skin cells expressed tyrosine hydroxylase, a marker for peripheral catecholaminergic neurons that is not detectable in embryonic skin (Fig. 3B). The number of neurons generated in these experiments was much higher from embryonic than adult skin.

We then asked when SKPs could first be isolated from skin. Initially, we confirmed that at low cell densities, one primary SKPs-forming cell gave rise to a single, clonal SKPs sphere. Three types of evidence supported this conclusion. First, at low densities (25,000 to 100,000 cells/ml with E18 skin cells), increasing the primary cell number increased the number of SKP spheres in a linear fashion (Fig. 10b). Second, when YFP-positive and unlabelled skin cells were mixed, the YFP-positive and negative spheres were mutually exclusive (Fig. 2F). Third, when immobilized as individual cells in methylcellulose, primary skin cells generated FGF2- and EGF-dependent spheres expressing the SKPs markers nestin, fibronectin (Fig. 3C), and Sca-1. When replated in methylcellulose, single cells from primary spheres generated secondary spheres expressing the same SKPs markers, indicative of self-renewal. Importantly, similar numbers of SKP spheres were obtained using methylcellulose and low-density liquid cultures, confirming the clonality of spheres obtained in liquid cultures.

Using these assays, we quantified the number of SKPs-like precursors in skin (Fig. 3D, Fig. 10C), and demonstrated that SKP spheres were never generated from mouse skin prior to E14. From E15 to E19 there was a burst of SKPs-forming cells in skin which peaked at 0.5 to 1% of skin cells, and then decreased approximately 10-fold by P0. While the frequency of sphere-forming cells apparently decreased further into adulthood, their actual number was similar in back skin at P0 and in adulthood (Fig. 10C).

We then differentiated clonally-derived primary embryonic spheres (Fig. 10D) and asked whether they, like passaged SKPs, differentiated into neural and mesodermal progeny. Double-label immunocytochemistry for SMA and βIII-tubulin revealed that 100% of these clones generated SMA-positive cells, and 75% also generated βIII-tubulin-positive, SMA-negative neurons (Fig. 3E). Analysis for SMA and GFAP revealed that, of these clones, 100% generated SMA-positive cells, and 15% also generated GFAP-positive, SMA-negative glial cells (Fig. 3E). Similar results were obtained with primary spheres from methylcellulose cultures (Fig. 10E). Thus, after E14.5, embryonic mouse skin contains precursor cells that can generate neurons, and embryonic primary clonal spheres exhibit a differentiation potential similar to passaged SKPs.

We also performed two sets of experiments which indicated that SKPs did not arise by transdifferentiation or dedifferentiation of Schwann cells or melanoblasts, both neural crest-derived cell types that are abundant in skin. First, analysis of primary, unpassaged SKP spheres from neonatal skin revealed that they did not contain cells expressing the melanoblast/melanocyte markers trp1, c-kit, or dct (Fig. 10G), or the Schwann cell markers MBP, P0, p75NTR (Fig. 1d) or Sox10. Second, we have recently found that when primary mouse skin cells are FACs-sorted for Sca-1, all of the SKPs sphere-forming ability is found within the Sca-1-positive population (KMS, DRK and FDM, unpublished data). However, immunocytochemical analysis of primary dissociated skin cells for Sca-1 and the melanoblast/melanocyte markers dct, c-kit and trp1, or the Schwann cell markers MBP, GFAP, and CNPase (Fig. 10H) demonstrated that neither melanoblast/melanocytes nor Schwann cells were Sca-1-positive.

### Example 3: Dermal papillae of hair and whisker follicles are one niche for endogenous SKPs

Since SKPs express a distinctive panel of embryonic transcription factors, we asked whether they could be used to identify a niche for endogenous SKPs in vivo. To test this possibility, we first confirmed that mouse skin contained cells expressing nestin, snail and twist from embryogenesis to adulthood (Fig. 4U). We then performed in situ hybridization on back skin sections from E18 to adulthood (P36) (Figs. 4A-4T). This study revealed that slug, snail and twist mRNAs were all expressed in the hair follicle papilla, a dermally-derived structure located at the base of the follicle (Fig. 4A) that is thought to contain multipotent precursor cells. At E18, hair follicle papillae were characterized by robust alkaline phosphatase (AP) activity, expression of versican, a cell surface proteoglycan, and nexin, an anagen-specific matrix modifying factor (Figs. 4A, 4B, 4E, and 4F). Low-level expression of twist, slug, and snail (Figs. 4C, 4D) was also clearly detected in these developing structures. At postnatal day 2, most of the maturing follicles were approaching the first synchronized anagen growth phase of the hair cycle, and low level expression of snail, slug and twist could be detected in the papillae (Figs. 4G, 4I, 4J, and 4K), in addition to AP, nexin and versican (Figs. 4G, 4L, and 4M). Expression of these markers did not overlap with keratin 17 expression, a marker for the bulge epidermal stem cell niche and the outer root sheath (Fig. 4H) or with keratin 5, a marker for the basal epidermis (Fig. 4G). By P19, hair follicles were in the first synchronous telogen rest phase, and their associated papillae were small structures at the distal tip of resting follicles (Fig. 4N). These structures still expressed AP (Fig. 4N), but did not express the anagen markers nexin or versican (Figs. 4Q, 4R) or the transcription factors snail (Fig. 4O) or slug. Very low levels of twist were still detected (Fig. 4P). At postnatal day 36, during the next wave of anagen, twist (Figs. 4S, 4T), snail, and slug were all re-expressed, along with nexin and versican. Thus, the embryonic transcription factors slug, snail, and twist are coordinately expressed in papillae during the anagen growth phase of the hair cycle.

To ask whether follicle papillae represented an endogenous niche for SKPs, as these data suggested, we examined the larger whisker vibrissal papillae, which are amenable to micro dissection. At E14.5, when whisker follicles first form, the epidermal downgrowths are surrounded by dermal condensates that express AP (Fig. 5A). Expression of snail and twist was widespread at this stage (consistent with the neural crest origin of the facial dermis), but was highest in these dermal condensates (Figs. 5A-5C), as was expression of slug, versican, and nexin. In contrast, Wnt5a mRNA, which is enriched in papillae at later stages, was uniformly expressed in the dermis (Fig. 5D). By E16.5, the vibrissal papillae were distinct structures, and contained cells that expressed snail (Figs. 5F, 5J), slug (Figs. 5G, 5K), twist, nexin (Fig. 5M), versican (Fig. 5N), and Wnt5a (Figs. 5H, 5O). At this stage, the papillae also contained nestin-expressing cells (Fig. 5L). At E18.5, vibrissal papillae continued to express slug, nexin, and versican (Figs. 5R, 5S, 5T) in a pattern distinct from that of keratin 15 (Fig. 5Q). The expression of slug (Fig. 7B), snail, and twist persisted in the whisker papillae postnatally. Slug, snail and twist were also expressed in newly-formed hair follicle papillae in the E16.5 whisker pad, as seen in dorsal skin (Fig. 4).

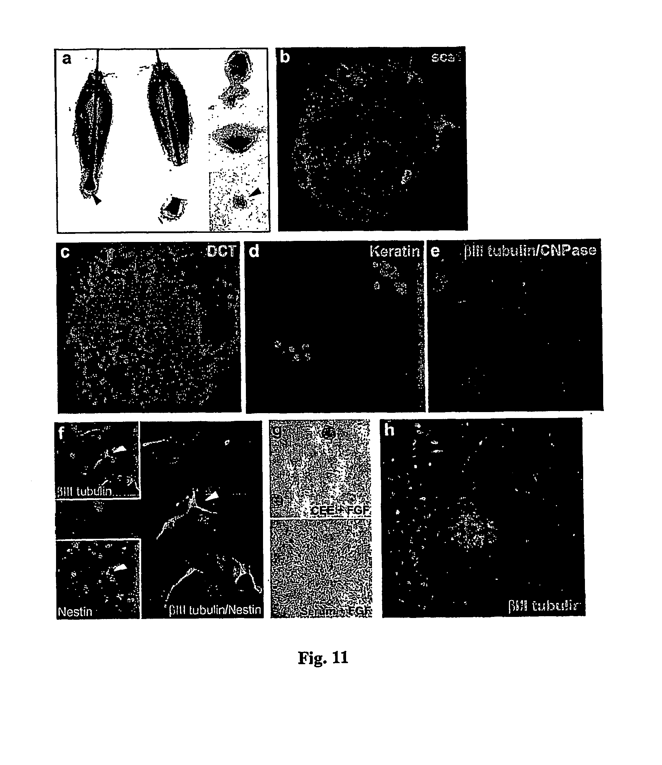

Consistent with the idea that follicle papillae are an endogenous niche for SKPs, RT-PCR demonstrated that neonatal SKPs but not CNS neurospheres expressed the papilla markers, nexin, versican, and Wnt5a (Fig. 6A). Moreover, immunostaining confirmed that most neonatal SKP cells were versican-positive (Fig. 6B). We then obtained two lines of evidence indicating whisker follicles contained SKPs-like cells. First, immunostaining of dissociated, differentiated whisker cells revealed a subpopulation of nestin- and βIII-tubulin-positive cells with neuronal morphology. Second, YFP-positive adult mouse vibrissal cells generated SKPs-like spheres when cocultured with unlabelled E16 skin cells (Fig. 10F). We therefore asked if dissected vibrissal papillae (Fig. 11A) contained SKP-like cells, after confirming that they did not contain melanoblasts/melanocytes, Schwann cells, or βIII-tubulin-positive neurons, as indicated by the following evidence. First, dissected papillae contained Sca-1-positive cells, but not dct-positive cells (Fig. 11B, 11C). Second, dissociated papillae cells did not express keratin 18 (a marker for Merkel cells), CNPase, or βIII-tubulin (Figs. 11D, 11E). We then determined whether dissected papillae contained cells with SKPs-like potential. As seen for total vibrissal cells, differentiation of dissociated papilla cells generated a subpopulation of nestin- and βIII-tubulin-positive cells with the morphology of immature neurons (Fig. 11F). Moreover, when cultured under SKPs conditions, dissociated papilla cells generated spheres (Fig. 11G) that expressed versican (Fig. 6C), nestin, and fibronectin (Fig. 6D). Finally, differentiated papillae spheres generated cells that expressed βIII-tubulin or SMA with morphologies similar to the young neurons and smooth muscle cells produced by SKPs (Figs. 6E, 6F, Fig. 11h).

Thus, three lines of evidence argue that hair and whisker follicle papillae are endogenous niches for SKPs; (i) follicle papillae contain cells that express the same embryonic transcription factors as do SKPs, (ii) SKPs express markers specific for follicle papillae in skin, and (iii) postnatal vibrissal papillae contain cells that proliferate as nestin-positive SKP spheres, and that differentiate into cells with attributes of neurons and smooth muscle cells.

### Example 4: Follicle dermal papillae contains neural crest-derived cells, and SKPs from facial skin are neural crest-derived

The above findings argue that SKPs are a neural crest-related precursor that arises in dermis during embryogenesis and persists into adulthood, and that follicle papillae are niches for these precursors. These findings predict that follicle papillae, whose embryonic origin is currently unknown, would contain neural crest-derived cells, and that SKPs themselves would be neural crest-derived. To test these predictions, we utilized a genetic method previously used to "tag" neural crest-derived cells in vivo. Specifically, mice expressing a Wnt1-Cre transgene were crossed to those expressing a floxed RosaR26R reporter allele, thereby marking the progeny of NCSCs with β-galactosidase. Using this approach, we first asked whether hair follicle papillae were neural crest-derived; serial sections of dorsal skin from Wnt1Cre;RosaR26R animals were analyzed for β-galactosidase by in situ hybridization (Fig. 7A, Fig. 12A). At P5, when all pelage follicles enter the first synchronized anagen phase, we observed β-galactosidase-positive cells in the follicle papillae (Fig. 7A) and occasional cells in the outer dermal follicle sheath (Fig. 12A). These β-galactosidase-positive cells did not express keratin 15. Faintly β-galactosidase-positive cells were also occasionally found in the epithelial compartment of the hair follicle unit, including a few cells in the matrix (Fig. 7A), bulge region (data not shown) and exiting to the interfollicular epidermis (Fig. 12A). These latter β-galactosidase-positive cells are likely progeny of NCSC-derived melanoblast stem cells previously reported to inhabit these niches, and/or may represent other NCSC-derived progeny such as Merkel cells or Schwann cells. β-galactosidase-positive cells were also identified in follicle papillae of adult mice. Thus, hair follicle dermal papillae contain neural crest-derived cells. However, low transgene penetrance in dorsal skin made it difficult to assess the precise contribution made by neural crest to this follicle compartment (Figs. 12B, 12C).

We analyzed the developing whisker pad of Wnt1Cre;R26R mice. At postnatal day 9, a time when SKP-like spheres can be isolated from papillae (Fig. 6), almost all whisker papillae cells expressed the β-galactosidase transgene, as they did nexin and slug (Fig. 7B). The vast majority of dissected neonatal whisker papilla cells also stained positive for β-galactosidase activity (Fig. 7D). Similarly, at E18.5, almost all cells in newly-formed papillae (Fig. 7C), and many of the dermal cells were β-galactosidase positive, consistent with the neural crest origin of facial dermis. Thus, vibrissal papillae are almost entirely of neural crest origin.

We then asked whether SKPs are neural crest-derived. SKP spheres generated from neonatal Wnt1Cre;Rosa26 whisker pads and stained with X-gal after 0 to 2 passages were all β-galactosidase-positive (Fig. 8A). When individual transgene-positive spheres from whisker pads were differentiated for two weeks, they differentiated into SMA-positive smooth muscle cells and βIII-tubulin-positive neurons (Fig. 8B), demonstrating SKP-like potential. While the transgene was downregulated in most cells during differentiation, occasional β-galactosidase-positive smooth muscle cells and neurons were still observed (Figs. 8C, 8D). Thus, SKPs generated from facial skin are neural crest-derived. These findings, combined with our data showing that whisker follicle papillae are neural crest-derived, that follicle papillae express many genes in common with SKPs, and that isolated vibrissal papillae generate SKP-like spheres, strongly support the idea that hair and whisker follicle papillae represent endogenous niches for these adult neural crest-related precursor cells.

The above experiments described in Examples 1-4 were carried out using the following methods and materials.

### Methods

### Cell culture

SKPs were cultured as described in Toma et al. (Nat. Cell Biol. 3:778-784, 2001). Briefly, dorsal or facial skin from mouse embryos (E15-19), mouse or rat neonates (P2-P6) or adults (3 weeks and older) was dissected from the animal and cut into 2-3 mm² pieces. Tissue was digested with 0.1% trypsin for 10-45 min at 37°C, mechanically dissociated and filtered through a 40 µm cell strainer (Falcon). Dissociated cells were pelleted and plated in DMEM-F12, 3:1 (Invitrogen), containing 20 ng/ml EGF and 40 ng/ml FGF2 (both Collaborative Research), hereafter referred to as proliferation medium. Cells were cultured in 25 cm² tissue culture flasks (Falcon) in a 37°C, 5% CO2 tissue culture incubator. SKPs were passaged by mechanically dissociating spheres and splitting 1:3 with 75% new medium and 25% conditioned medium from the initial flask. Neurospheres from the E13 embryonic telencephalon were cultured under the same conditions. For neuronal differentiation, SKP spheres or primary dissociated skin cells were mechanically dissociated and plated on chamber slides (Nunc) coated with poly-D-lysine/laminin in DMEM-F12 3:1 supplemented with 40 ng/ml FGF2 and 10% FBS (BioWhittaker) for 5-7 days. Cells were then cultured an additional 5-7 days in the same medium without FGF2 but with the addition of 10 ng/ml NGF (Cedar Lane), 10 ng/ml BDNF (Peprotech) and 10 ng/ml NT3 (Peprotech). For Schwann cell differentiation, dissociated spheres were cultured in DMEM-F12 3:1 supplemented with 10% FBS for 7 days, then switched to the same medium supplemented with 4 µM forskolin (Sigma).

For the vibrissae experiments, rat vibrissal follicles were dissected from P6 to P21 whisker pads and excess tissue was carefully removed. In some experiments (such as Sca-1 immunostaining) papillae were dissected from mice of a similar age. The inner root sheath was opened with tungsten needles and the papilla removed. Papillae were digested with trypsin for 15 min at room temperature and mechanically dissociated. Single cells were plated on 2-well chamber slides coated with poly-D-lysine/laminin/fibronectin and cultured using the neuronal differentiation protocol described above. Alternatively, total vibrissal cells were dissociated and treated in the same way. To generate spheres from isolated papilla cells, cells were plated on chamber slides coated with poly-D-lysin/laminin/fibronectin in SKPs proliferation medium supplemented with 5% chick embryo extract. After 7 days, adhered spheres were removed from the slide and a single sphere was then replated in a new chamber slide for differentiation using the neuronal differentiation protocol described above.

Sphere counts in solution were performed after seeding 25,000 - 200,000 cells/ml in uncoated 24-well tissue culture plates (Falcon) in proliferation medium for 4-7 days. Methylcellulose sphere counts were performed by plating dissociated, individual skin cells (100,000) in DMEM-F12 (3:1), 1.5% methylcellulose (Sigma), 2% B27 (Gibco-BRL), 20 ng/ml EGF and 40 ng/ml FGF2, 1 µg/ml fungizone (Invitrogen) and 1% penicillin/streptomycin. Cells were cultured in 3.5 cm plates in a 37°C, 5% CO₂ incubator and sphere formation was scored after 10-14 days. For passaging, individual spheres were picked from the methylcellulose, dissociated to single cells, and replated again in methylcellulose. Cell mixing experiments were performed by mixing dissociated E16 or E18 skin cells with YFP-tagged dissociated vibrissal follicle cells 100:1 in uncoated flasks with proliferation medium at 25,000 cells/ml.

### Immunocytochemistry, in situ hybridization, and X-gal staining

Immunocytochemical analysis for cells was performed either using coated slides and the cytospin system (Thermo Shandon) for SKP spheres, or on cells plated on chamber slides (Nunc) as previously described (Toma et al., Nat. Cell Biol. 3:778-784, 2001; Barnabé-Heider et al., J. Neurosci. 23:5149-5160, 2003). The following primary antibodies were used: anti-nestin monoclonal, 1:400 (BD Biosciences), anti-βIII-tubulin monoclonal, 1:500 (Tuj1 clone; BABCO), anti-neurofilament-M polyclonal, 1:200 (Chemicon), anti-GFAP polyclonal, 1:200 (DAKO), anti-p75NTR polyclonal, 1:500 (Promega), anti-SMA monoclonal, 1:400 (Sigma), anti-fibronectin polyclonal, 1:400 (Sigma), anti-trp1 polyclonal, 1:200 (Chemicon), anti-c-kit polyclonal (Cell Signaling Technology), anti-S100β monoclonal, 1:1000 (Sigma), anti-MBP polyclonal, 1:100 (Chemicon), anti-TH monoclonal, 1:200 (Chemicon), and Sca-1, 1:100 (Becton Dickinson). Secondary antibodies used were: Alexa488-conjugated goat anti-mouse, 1:1000 and Alexa594-conjugated goat anti-rabbit, 1:1000 (both from Molecular Probes). Processing of skin samples for histological analysis and in situ hybridization was performed as described in Mo et al. (Development 124:113-123, 1997). The probes used in this study were as follows: β-galactosidase (Ambion); nexin and versican (kind gifts from Dr. Bruce Morgan); K17 (P. Coulombe); Wnt5a (A. McMahon); Snail (T. Gridley); Slug, twist and nestin probes were all generated using the RT-PCR primers detailed below. Immunohistochemistry and alkaline phosphatase staining on skin sections was performed as previously described (Mill et al., Genes Dev. 17:282-294, 2003). Briefly, staining for lacZ was carried out on tissues fixed in 2.7% formaldehyde, 0.02% Nonidet-P40, PBS overnight at 4°C, followed by overnight cryoprotection in 30% sucrose in PBS at 4°C prior to mounting. Cryosections were cut at 12 µm thickness and stained overnight at 37°C in X-gal staining solution. Sections were counterstained in eosin and mounted. Plated cells and spheres were X-gal stained by briefly fixing in 4% formaldehyde (2 min), rinsing three times in PBS, rinsing twice in 0.1M sodium phosphate containing 2 mM MgCl2, 0.1 % sodium desoxycholate, and 0.02% NP40, and then immersing in standard X-gal staining solution overnight.

### RT-PCR

RNA was prepared from samples using Trizol (Invitrogen), and cDNA was generated with Revertaid Reverse Transcriptase (Fermentas) as directed by the manufacturer. For all cDNA synthesis, a -RT control was performed. PCR reactions were carried out as follows: 92°C 2 min., 30-35 cycles of 94°C for 60 s., Gene-specific annealing temperature for 60 s. and 72°C for 60 s. PCR primers used were as follows:

| Gene | Primer Sequence | Anneal Temp (°C) | Product (BP) | SEQ ID NO. |
|---|---|---|---|---|
| Pax3 | ggaggcggatctagaaaggaagga | 59 | 374 | 1 |
| | cccccggaatgagatggttgaa | | | 2 |
| Slug | cgtcggcagctccactccactctc | 60 | 348 | 3 |
| | tcttcagggcacccaggctcacat | | | 4 |
| Twist | cgccccgctcctctgctctaccc | 65 | 352 | 5 |
| | gccgccgccgccaccacctc | | | 6 |
| Snail | cggcgccgtcgtccttct | 61.5 | 398 | 7 |
| | ggcctggcactggtatctcttcac | | | 8 |
| Sox9 | ccgcccatcacccgctcgcaatac | 59.5 | 544 | 9 |
| | gcccctcctcgctgatactggtg | | | 10 |
| P75 | gtgcggggtgggctcaggact | 62 | 422 | 11 |
| | ccacaaggcccacaaccacagc | | | 12 |
| SHOX2 | ccgccgccgccaagaccac | 63 | 355 | 13 |
| | tccccaaacccgctcctacaaa | | | 14 |
| DBH | acccgggggacgtactcatcac | 59 | 353 | 15 |
| | cgggaagcggacagcagaag | | | 16 |
| Peripherin | gccgccaaccgcaaccat | 61.5 | 333 | 17 |
| | gatccggctctcctccccttcc | | | 18 |
| MBP | tggccccggggacacttc | 61.1 | 332 | 19 |
| | gccgctgaccaccccaccat | | | 20 |
| P0 | cctggctgccctgctcttctcttc | 60.1 | 452 | 21 |
| | ccccgatcactgctcccaacac | | | 22 |
| Dermo-1 | gcggcgctacagcaagaaatc | 61.4 | 356 | 23 |
| | ccatgcgccacacggagaagg | | | 24 |
| Nexin | ccacgcaaagccaagacgac | 57.4 | 289 | 25 |
| | gaaaccggcctgctcatcct | | | 26 |
| Versican | tggaaggcacagcagtttacc | 56.3 | 427 | 27 |
| | tcatggcccacacgattcac | | | 28 |
| Wnt-5a | ccccctcgccatgaagaagc | 60.1 | 552 | 29 |
| | cagccgccccacaaccagt | | | 30 |
| GAPDH | gtcttcaccaccatggagaag | 56 | 281 | 31 |
| | gtgatggcatggactgtggtc | | | 32 |

### Western blot analysis

Lysates were prepared and western blot analysis performed as described previously (Barnabé-Heider et al., J. Neurosci. 23:5149-5160, 2003). Equal amounts of protein (50-100 µg) were analyzed on 7.5% or 10.5% polyacrylamide gels. The primary antibodies used were anti-DβH monoclonal 1:1000 (Pharmingen), anti-peripherin polyclonal, 1:1000 (Chemicon), anti-p75NTR polyclonal, 1:1000 (Promega), anti-TH monoclonal, 1:800 (Chemicon), anti-βIII-tubulin monoclonal, 1:1000 (Tuj1 clone; BABCO), anti-NCAM monoclonal, 1:800 (Chemicon).

### In ovo transplantations

Fertile White Leghorn chicken eggs (Cox Brothers Poultry Farm, Truro, NS) were incubated at 37°C in a humidified chamber until Hamburger and Hamilton (HH) stage 18. A small opening was made in the side of the shell and a small bolus of neutral red/water solution (1% w/v) was applied to the chorioallantoic membrane to visualize the underlying embryo. An incision was made into the anterior, medial corner of two somites in the lumbar region for each embryo using needles made from flame-sharpened tungsten wire. One or two SKP neurospheres (-200-250 total cells) were transplanted into the incision site that corresponds to the dorsal most region of the neural crest migratory pathway. The shells were subsequently sealed and the embryos incubated until HH stage 30 (- 3 more days) after which time the embryos were removed from the shell contents, quickly decapitated, eviscerated, fixed in 3.7% formaldehyde/PBS for 2 hours, and immersed in 30% sucrose/PBS overnight at 4°C. The embryos were embedded in OCT, frozen at - 70°C, sectioned at 20µm and mounted onto tissue-adhering slides.

### Example 5: SKPs differentiate into Schwann cells in vitro

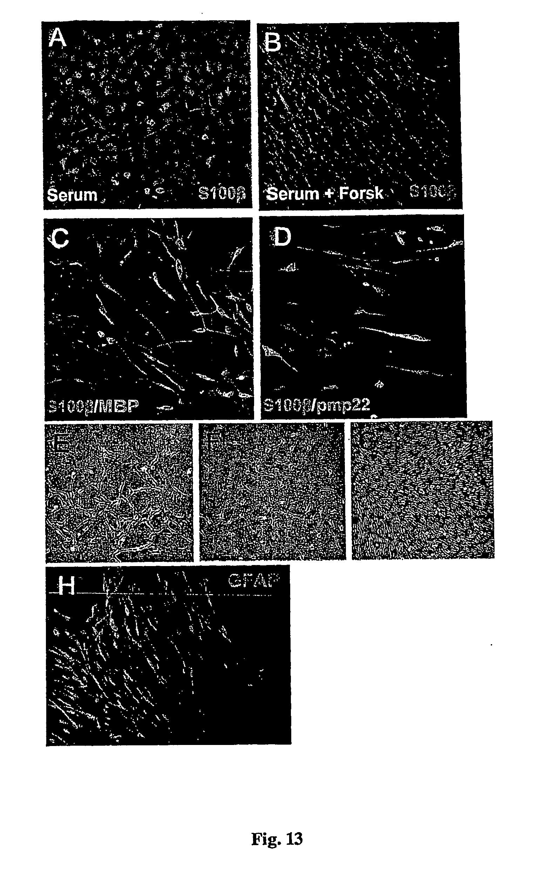

We previously found that a small proportion of clonally derived SKPs spheres differentiated in serum with FGF2 followed by serum and neurotrophic growth factors generate Schwann cells. The elevation of cAMP levels by the addition of forskolin has been shown to potentiate the differentiation of Schwann cells from neural tube-derived neural crest stem cells and induce the expression of myelin proteins. Differentiation of SKPs in media supplemented with serum and forskolin leads to the generation of parallel arrays of S100β positive cells, characteristic of Schwann cells (Fig. 13B). Few S100β positive cells are found in cultures differentiated in serum alone (Fig. 13A). Further characterization of these cells revealed that they coexpress the Schwann cell marker S100bwith the myelin proteins MBP or pmp22 (Figs. 13C, 13D). While the addition of forskolin potentiates Schwann cell differentiation from SKPs in the presence of serum, Schwann cells represent only 20-30% of the total number of cells. We sought to identify conditions under which Schwann cells could be generated with greater efficiency to facilitate analysis of their myelination potential and to yield a Schwann cell source for transplantations.

### Example 6: SKP-derived Schwann cells (SKP-SCs) can be isolated under serum-free conditions

Heregulin-β of the neuregulin family of growth factors is critical for Schwann cell differentiation during development and from neural crest stem cells in vitro. When single, clonal SKPs spheres are differentiated in serum-free media supplemented with N2, heregulin-β and forksolin, 83% (80/96) of differentiated cultures contained GFAP-positive Schwann cells (Fig. 13H), compared to just 3% (3/96) of cultures differentiated in media supplemented with N2 alone. The differentiation of SKPs in these serum-free conditions had the added benefit of minimizing the proliferation of smooth muscle cells that propagate readily in media containing serum. Small colonies of SKP-derived Schwann cells could be readily observed under phase microscopy (Fig. 13E) and these colonies were isolated with cloning cylinders and replated in new dishes (Fig. 13F). Schwann cells selected in this way could be expanded to yield cultures of greater than 95% purity (Fig. 13 G).

### Example 7: SKP-SCs associate with and myelinate DRG neuron axons in vitro

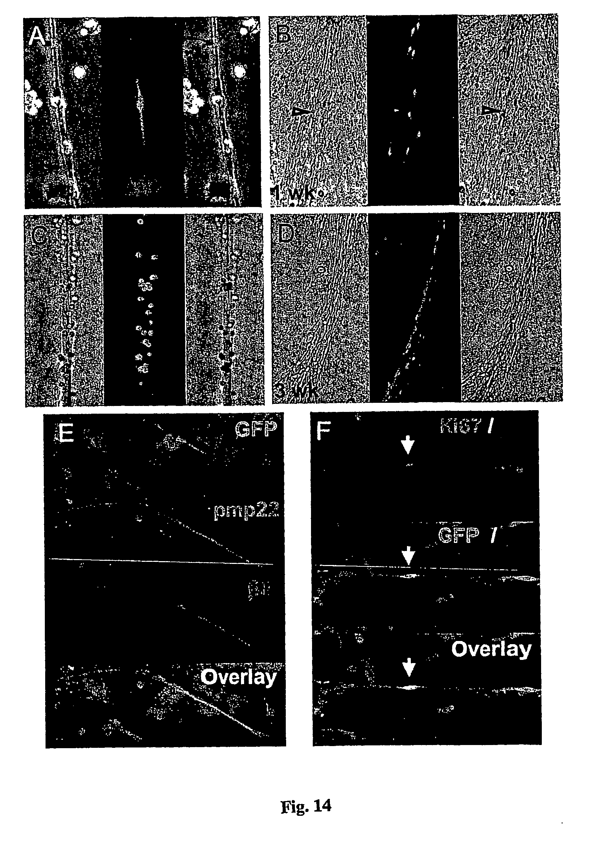

To assess the functionality of SKP-SCs, we employed the use of a DRG-neurons co-culture system. To distinguish SKP-SCs from endogenous Schwann cells that contaminate DRG-neuron cultures, we employed the use of SKPs cultured from YFP-transgenic mice. YFP-tagged SKP-SCs could be observed associating with neuronal processes (Figs. 14A, 14B). After 1 week, SKP-SCs associated with neuronal processes expressed Ki67 (Fig. 14F), a marker of proliferation and were observed to populate those processes (Figs. 14C, 14D). Given that the co-cultures were maintained in media devoid of Schwann cell mitogens, it can be concluded that SKP-SCs proliferated in response to axon-derived signals. Co-cultures were then maintained for a further 2 weeks with addition of ascorbic acid and IGF-1 to the media, factors that induce myelination in vitro. Under these conditions, pmp22-positive myelinated fibers comprised of YFP-positive SKP-SCs were observed (Fig. 14E).

### Example 8: SKP-SCs survive transplantation into organotypic cerebellar slice and express myelin proteins.

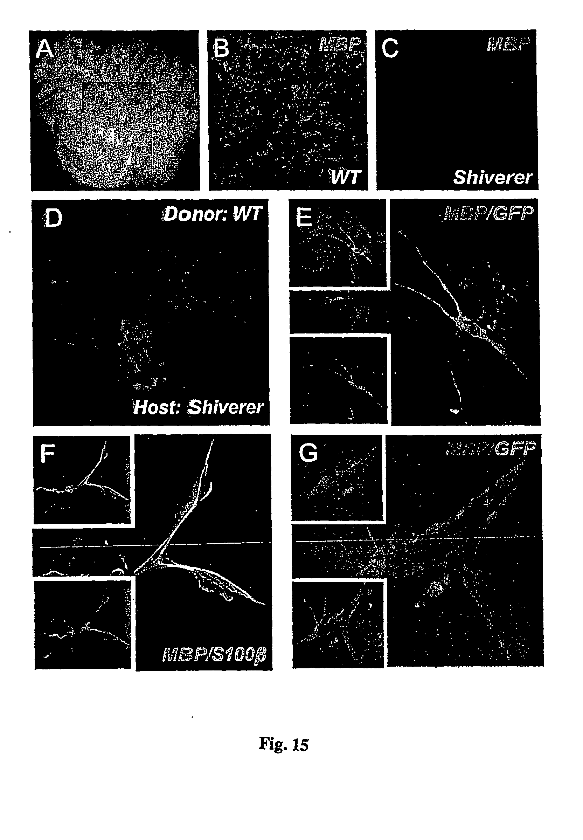

To determine the ability of SKP-SCs to survive in a CNS environment, we performed transplantations into the white matter tracts of cerebellar organotypic slice cultures. In order to reliably identify transplanted cells, we cultured cerebellar slices from shiverer mice (Fig. 15A), which have no endogenous MBP expression due to an autosomal recessive mutation (Figs. 15B, 15C). Transplanted cells cultured from wild type animals can be identified in this system by their expression of MBP (Fig. 15D). To validate this system we transplanted SKP-SCs cultured from YFP-transgenic mice, and found that expression of MBP was limited to transplanted cells (Fig. 15E, 15G). SKP-SCs were transplanted into the white matter tracts of slices. Two weeks after transplantation, YFP-positive cells that also expressed MBP were observed. After tranplantation of non-tagged SKP-SCs into shiverer slices, cells that co-expressed s100β and MBP with differentiated membranous processes were present in the slices (Fig. 15F). Long MBP and YFP-positive fiber tracts were formed in the white matter tracts of shiverer slices (Fig. 15G).

### Example 9: SKP-SCs integrate into sciatic nerve after transplantation and express myelin proteins

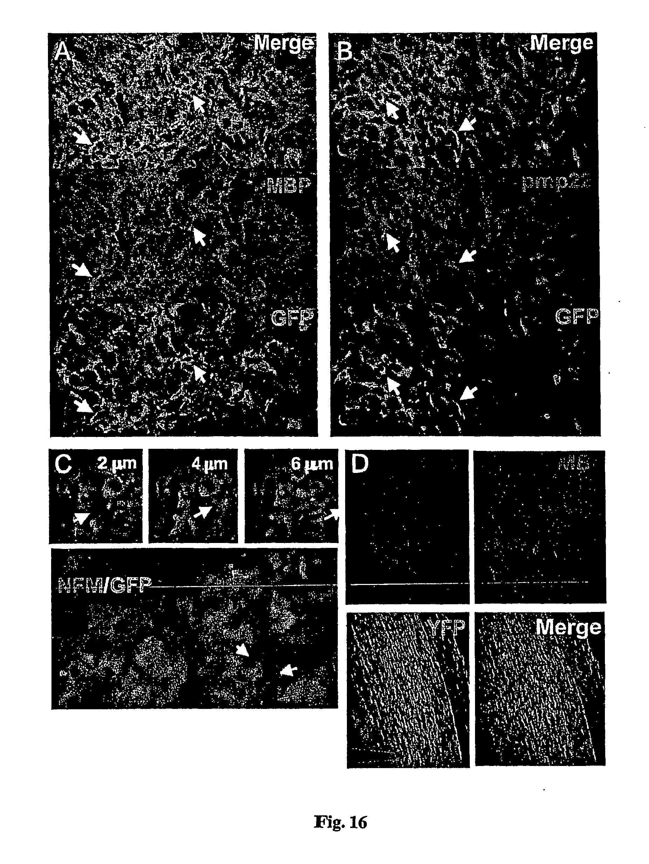

To assess the ability of SKP-SCs to myelinate the peripheral nervous system, we performed transplantations into the sciatic nerve. YFP-tagged SKP-SCs were transplanted into un-injured sciatic nerves, proximal to the bifurcation of the nerve. After 1-2 weeks, transplanted cells had migrated proximally and distally from the site of transplantation and were aligned longitudinally in the nerve (Fig. 16D). Although cells could be observed in close proximity to axons, few transplanted cells expressed myelin proteins. To increase the ability of the transplanted cells to associate with and myelinate axons in the nerve, we performed a crush injury prior to transplantation. SKP-SCs were injected distal to the crush injury such that regenerating axons would encounter the transplanted cells. In this model, transplanted cells survived in the nerve for up to four weeks, the longest time point examined. Transverse sections were stained with myelin protein antibodies and analysis by confocal microscopy revealed that the SKP-SCs expressed MBP and pmp22 with a morphology consistent with the profile of myelinated axons (Figs. 16A, 16B). YFP-tagged transplanted cells could be observed engulfing neurofilament positive axons over a series of adjacent optical slices (Fig. 16C).

### Example 10: SKPs integrate into the inferior colliculus of shiverer mice, produce myelin, and wrap axons

5x10⁵ cells from dissociated YFP-tagged SKPs spheres were injected into the inferior colliculus of postnatal day 1 shiverer mouse pups using a Hamilton syringe with a 30-gauge needle. After four weeks, animals were perfused with cold 4% formaldehyde, 2% glutaraldehyde in phosphate buffer. The brains were removed and 300 µm sections were made with a tissue chopper. Sections with YFP-positive transplanted cells were identified on an inverted fluorescent microscope. These sections were then processed for standard electron microscopy.

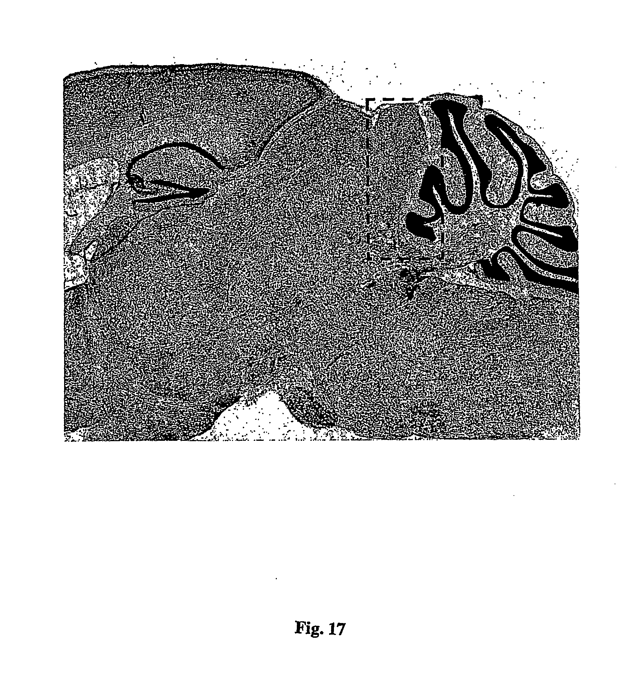

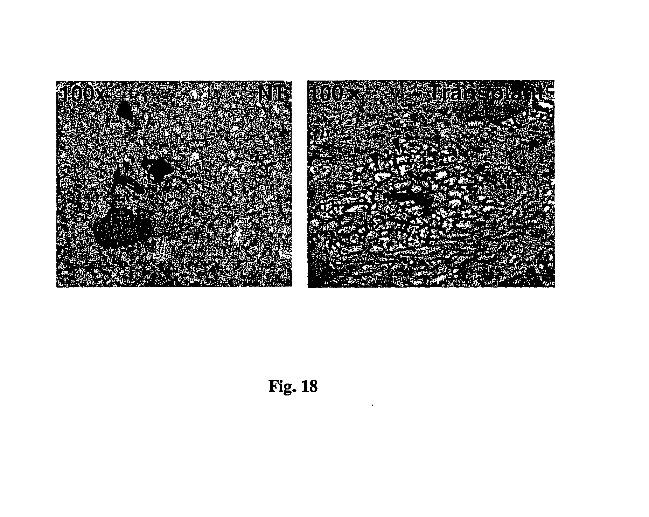

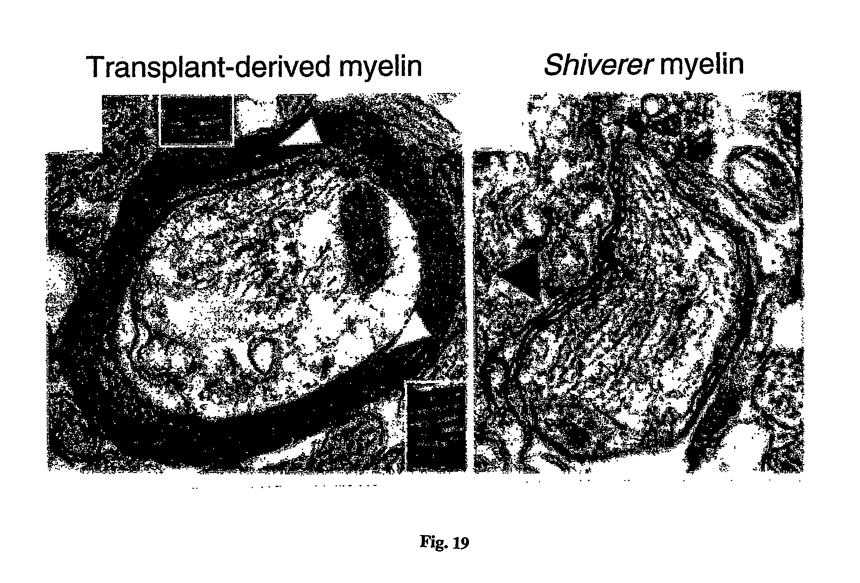

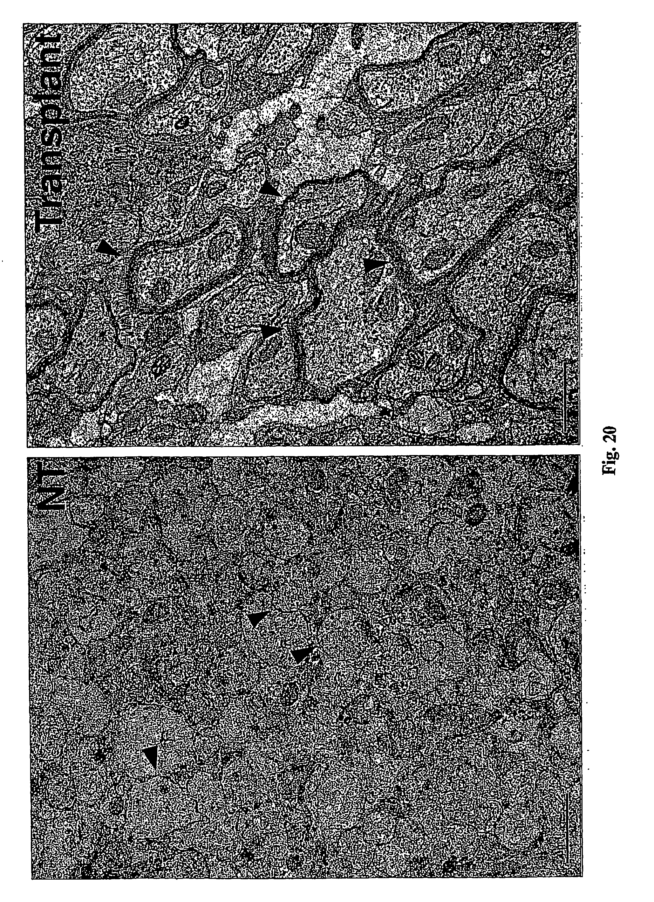

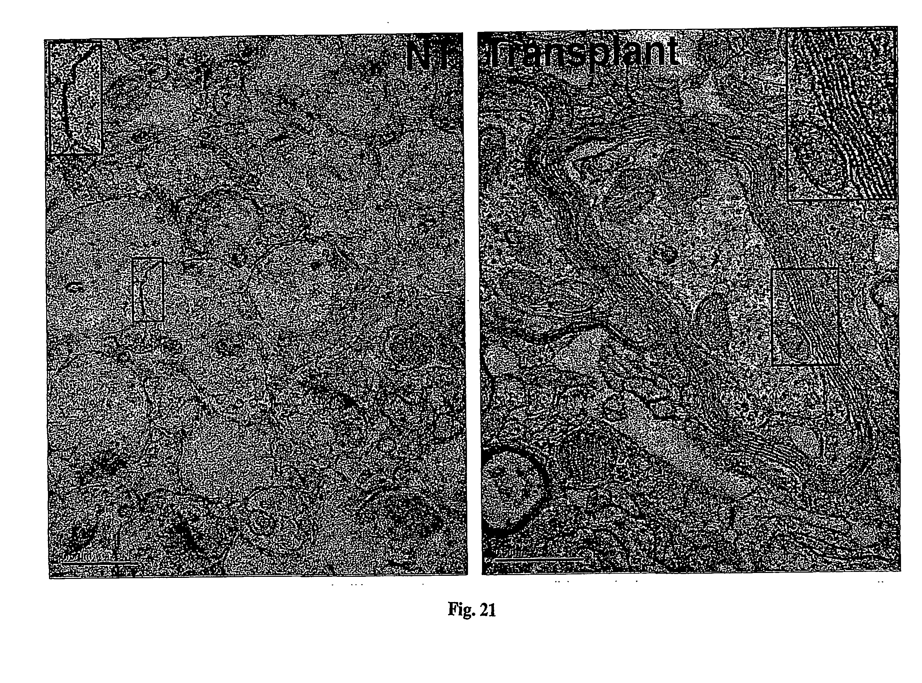

An image from a mouse-brain atlas is shown in Fig. 17 to illustrate the transplant site. The box indicates the inferior colliculus. Fig. 18 depicts semithin sections (1 µm) stained with toluidine blue. At 100x magnification on a light microscope darkly stained myelin profiles can be observed (arrow heads) in the transplanted brain (right panel) but not the non-transplanted shiverer brain control (left panel). Examples of normal myelin (left panel) and shiverer myelin (right panel) are shown in Fig. 19. Figs. 20 and 21 show electron microscopy demonstrates compact myelin wrapping host axons (arrow heads) of in the transplanted brain (right panel) but not the non-transplanted shiverer brain control (left panel). The inset in Fig. 21 shows the repeating major dense lines characteristic of compact myelin.

### Example 11: Transplanted SKPs migrate into hair follicles and express dermal papillae and dermal sheath markers

Postnatal (P1-3) SKPs were grown at low density as floating spheres. Adult NOD SCID mice (immunocompromised) were given two 6 mm wide wounds through the depth of the back skin. Three days following the injury, SKPs were dissociated and then injected (∼100,000 cells each) into the skin surrounding the wound. Animals were then sacrificed after two weeks and regenerated skin tissues processed for immunohistochemistry.

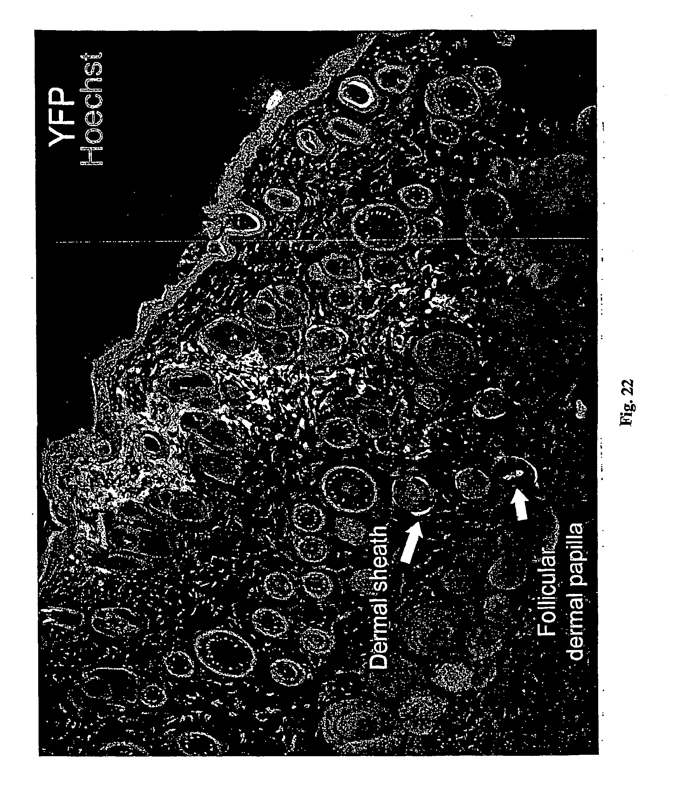

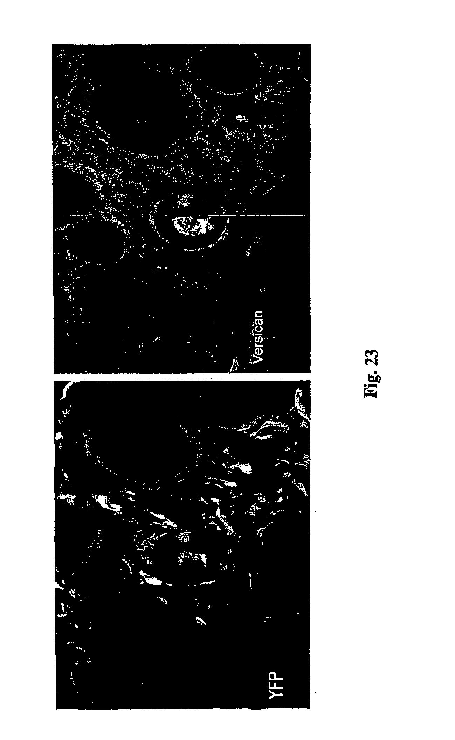

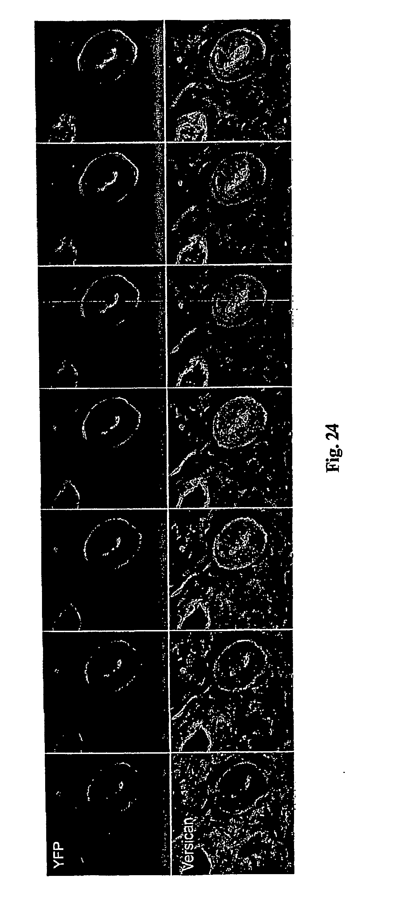

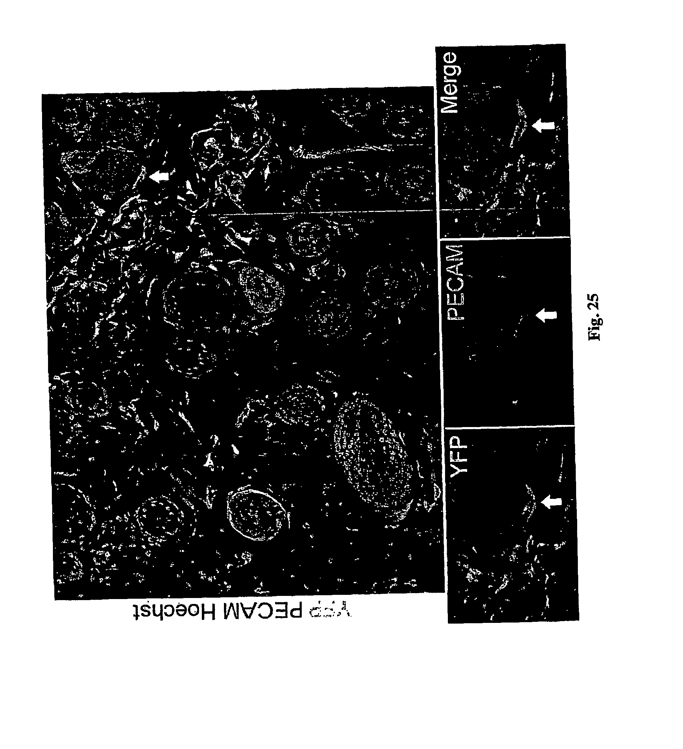

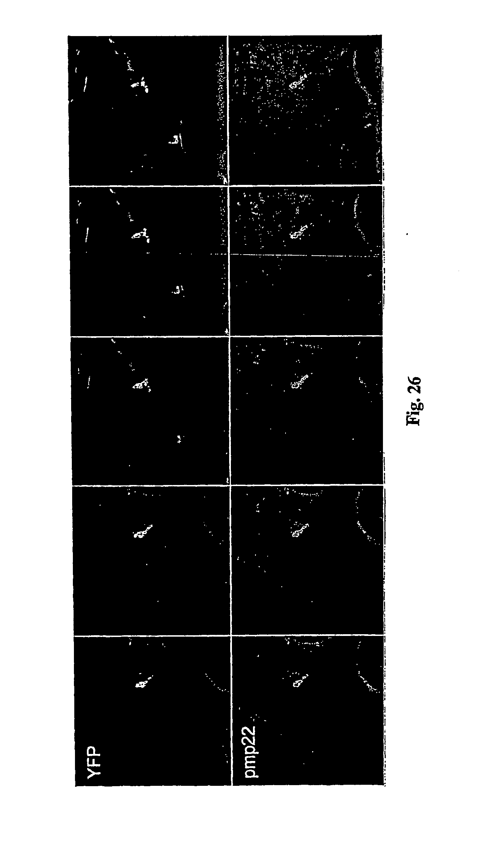

SKPs integrate into the epidermis and dermis, and into specialized structures such as the follicular dermal papillae and dermal sheath (Fig. 22). Moreover, SKPs express markers of follicular papillae (Fig. 23). Colocalization of SKPs in follicular papillae with versican, a chondroitin sulfate proteoglycan highly expressed within the cells of the papillae, particularly during anagen phase of the hair cycle. Fig. 24 is a high magnification confocal laser scanning microscopy image demonstrating that SKPs express versican. Transplanted SKPs also express PECAM, a marker of endothelial cells lining blood vessels (Fig. 25). SKPs express markers of Schwann cells following transplant into the skin. Fig. 26 shows a SKP-derived cell found in the dermis that expresses pmp22, a marker of peripheral Schwann cells.

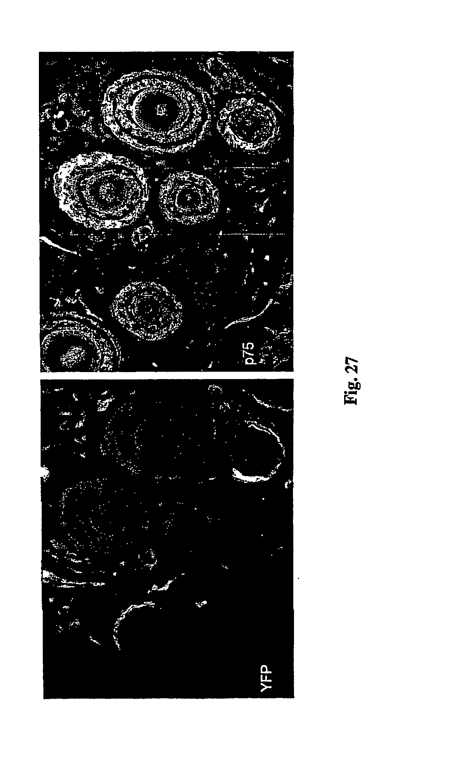

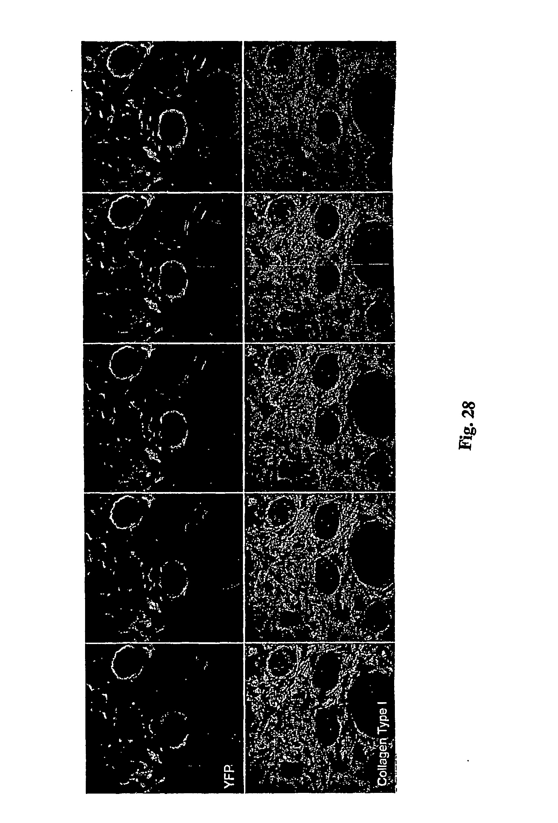

The dermal sheath is comprised of specialized fibroblasts and forms a collagen rich matrix. SKPs found within the dermal sheath low levels of p75 (Fig. 27), and also express collagen type I, which is expressed by endogenous dermal sheath cells (Fig. 28).

Dermal sheath and follicular papilla cells are of significance because of their close interaction with each other, their inherent ability to stimulate hair follicle formation, and induction of hair growth. The observation that SKPs are able to migrate into such a specialized structure (the hair follicle) and expresses markers consistent with those of dermal papillae and dermal sheath, demonstrate that SKPs may be capable of inducing hair growth, as well as regenerating dermal components within the adult mammalian skin (particularly following injury). SKPs transplantation may be an alternative or complement to skin grafts following severe burn, or mechanical injury to the skin, as well as for cosmetic purposes such hair replacement.

### Example 12: Isolation of human stem cells from hair follicles and skin

Multipotent stem cells can be purified from human hair follicles and human skin using the same procedures as described for the purification of stem cells from rodent hair follicles (as described herein) and rodent skin (as described in U.S. Patent Publication No. 2004-0033597). Source material is acquired by surgical removal of hair follicles or skin from the donor. Because the stem cells are capable of proliferation and self-renewal, little source tissue is required. Conditions for culturing human cells are described in U.S. Patent Publication No. 2004-0033597. Other conditions are known to those skilled in the art, and can be optimized for proliferation or differentiation of stem cells, if desired.

### Other Embodiments

The present invention has been described in terms of particular embodiments found or proposed by the present inventors to comprise preferred modes for the practice of the invention. It will be appreciated by those of skill in the art that, in light of the present disclosure, numerous modifications and changes can be made in the particular embodiments exemplified without departing from the intended scope of the invention. All such modifications are intended to be included within the scope of the appended claims.

All publications, patents and patent applications are herein incorporated by reference in their entirety to the same extent as if each individual publication, patent or patent application was specifically and individually indicated to be incorporated by reference in its entirety.

The following pages 37 to 45 contain specific embodiments.
1. A method of producing a population of at least ten cells, wherein at least 30% of said cells are multipotent stem cells or progeny thereof and wherein said multipotent stem cells are substantially purified from a hair follicle or a dermal papilla-containing portion thereof, said method comprising the steps of:
   (a) providing a hair follicle or a dermal papilla-containing portion thereof from a mammal;
   (b) culturing said hair follicle or said portion in conditions under which multipotent stem cells grow and proliferate non-adherently and at least 25% of the cells that are not multipotent stem cells die or adhere to the culture substrate; and
   (c) separating non-adherent cells from adherent cells and collecting non-adherent cells; and
   (d) continuing culture step (b) and (c) until at least 30% of collected cells are multipotent stem cells or progeny of said multipotent stem cells.
2. The method of 1, wherein said step (a) further comprises dissociating said hair follicle or said portion into smaller pieces by mechanical or enzymatic disruption.
3. A method of inducing hair growth in a mammal by providing to said mammal a population of cells, wherein at least 30% of said cells are multipotent stem cells or progeny thereof and wherein said multipotent stem cells are substantially purified from a hair follicle or a dermal papilla-containing portion thereof and are capable of producing hair follicle cells.
4. A method of inducing hair growth in a mammal by providing to said mammal a population of cells, wherein at least 30% of said cells are hair follicle cells that have differentiated from multipotent stem cells substantially purified from the dermal papillae of a hair follicle.
5. The method of any one of 1, 3, or 4, wherein at least 80% of the cells are multipotent stem cells substantially purified from said hair follicle or dermal papilla-containing portion thereof.
6. The method of 5, wherein at least 90% of the cells are multipotent stem cells substantially purified from said hair follicle or dermal papilla-containing portion thereof.
7. The method of 6, wherein at least 95% of the cells are multipotent stem cells substantially purified from said hair follicle or dermal papilla-containing portion thereof.
8. The method of any one of 1, 3, or 4, wherein said multipotent stem cells express at least one protein selected from the group consisting of nestin, WNT-1, vimentin, fibronectin, S100, slug, snail, twist, Pax3, Sox9, Dermo, and SHOX2.
9. The method of any one of 1, 3, or 4, wherein said multipotent stem cells do not express measurable levels of p75NTR.
10. The method of any one of 1, 3, or 4, wherein said multipotent stem cells do not express measurable levels of at least one protein selected from the group consisting of tyrosinase, c-kit, trypl, DCT, MBP, P0, and SOX10.
11. The method of any one of 1, 3, or 4, wherein said hair follicle is from an adult mammal.
12. The method of 9, wherein said hair follicle is from a juvenile mammal.
13. The method of any one of 1, 3, or 4, wherein said mammal is a human.
14. The method of any one of 1, 3, or 4, wherein said multipotent stem cells comprise cells that can differentiate into hair follicles cell under appropriate conditions.
15. The method of any one of 1, 3, or 4, wherein said multipotent stem cells comprise cells that can differentiate into neurons, astrocytes, Schwann cells, or oligodendrocytes under appropriate conditions.
16. The method of any one of 1, 3, or 4, wherein said multipotent stem cells comprise cells that can differentiate into non-neural cells.
17. The method of 14, wherein said non-neural cells are smooth muscle cells or adipocytes.
18. The method of any one of 1, 3, or 4, wherein said multipotent stem cell contains a heterologous gene in an expressible genetic construct.
19. The method of 16, wherein said gene encodes a therapeutic protein.
20. The method of 17, wherein said gene encodes a protein which induces or facilitates differentiation of said stem cell.
21. The method of 3 or 4, wherein said multipotent stem cells are obtained using the method of 1.
22. The method of 3 or 4, wherein said multipotent stem cells are from said mammal.
23. The method of 3 or 4, wherein said stem cells are from a donor mammal that is immunologically similar to said mammal.
24. The method of 3 or 4, wherein said mammal has a condition characterized by a reduced amount of hair.
25. The method of 24, wherein said condition is the result of alopecia, accidental injury, damage to hair follicles, surgical trauma, a burn wound, radiation therapy, chemotherapy, an incisional wound, or a donor site wound from skin transplant.
26. A kit comprising multipotent stem cells capable of inducing hair growth in a mammal and instructions for inducing hair growth in a mammal.
27. The kit of 26, wherein said stem cells are substantially purified from hair follicles or dermal papilla-containing portions thereof.
28. The kit of 26, wherein said multipotent stem cells express at least one protein selected from the group consisting of nestin, WNT-1, vimentin, fibronectin, S 100, slug, snail, twist, Pax3, Sox9, Dermo, and SHOX2.
29. The kit of 26, wherein said multipotent stem cells do not express measurable levels of p75NTR.
30. The kit of 26, wherein said multipotent stem cells do not express measurable levels of at least one protein selected from the group consisting of tyrosinase, c-kit, tryp1, DCT, MBP, P0, and SOX10.
31. The kit of 26, wherein said mammal has a condition characterized by a reduced amount of hair.
32. The kit of 26, wherein said condition is the result of alopecia, accidental injury, damage to hair follicles, surgical trauma, a burn wound, radiation therapy, chemotherapy, an incisional wound, or a donor site wound from skin transplant.
33. The kit of 26, wherein said mammal is a human.
34. A kit comprising multipotent stem cells capable of regenerating skin in a mammal and instructions for regenerating skin in a mammal.
35. The kit of 34, wherein said stem cells are substantially purified from hair follicles or dermal papilla-containing portions thereof.
36. The kit of 34, wherein said multipotent stem cells express at least one protein selected from the group consisting of nestin, WNT-1, vimentin, fibronectin, S 100, slug, snail, twist, Pax3, Sox9, Dermo, and SHOX2.
37. The kit of 34, wherein said multipotent stem cells do not express measurable levels of p75NTR.
38. The kit of 34, wherein said multipotent stem cells do not express measurable levels of at least one protein selected from the group consisting of tyrosinase, c-kit, tryp1, DCT, MBP, P0, and SOX10.
39. The kit of 34, wherein said mammal has a condition characterized by a reduced amount of hair.
40. The kit of 40, wherein said condition is the result of alopecia, accidental injury, damage to hair follicles, surgical trauma, a burn wound, radiation therapy, chemotherapy, an incisional wound, or a donor site wound from skin transplant.
41. The kit of 34, wherein said mammal is a human.
42. A method of inducing hair growth in a mammal by providing to said mammal a population of cells, wherein at least 30% of said cells are multipotent stem cells or progeny thereof and are capable of producing hair follicle cells.
43. The method of 42, wherein at least 80% of the cells are multipotent stem cells or progeny thereof and are capable of producing hair follicle cells.
44. The method of 43, wherein at least 90% of the cells are multipotent stem cells or progeny thereof and are capable of producing hair follicle cells.
45. The method of 44, wherein at least 95% of the cells are multipotent stem cells or progeny thereof and are capable of producing hair follicle cells.
46. The method of 42, wherein said stem cells are substantially purified from hair follicles or dermal papilla-containing portions thereof.
47. The method of 42, wherein said multipotent stem cells express at least one protein selected from the group consisting of nestin, WNT-1, vimentin, fibronectin, S100, slug, snail, twist, Pax3, Sox9, Dermo, and SHOX2.
48. The method of 42, wherein said multipotent stem cells do not express measurable levels of p75NTR.
49. The method of 42, wherein said multipotent stem cells do not express measurable levels of at least one protein selected from the group consisting of tyrosinase, c-kit, tryp1, DCT, MBP, P0, and SOX10.
50. The method of 42, wherein said mammal has a condition characterized by a reduced amount of hair.
51. The method of 50, wherein said condition is the result of alopecia, accidental injury, damage to hair follicles, surgical trauma, a burn wound, radiation therapy, chemotherapy, an incisional wound, or a donor site wound from skin transplant.
52. The method of 42, wherein said cells are from said mammal.
53. A method of regenerating skin in a mammal by providing to said mammal a population of cells, wherein at least 30% of said cells are multipotent stem cells or progeny thereof and are capable of regenerating skin.
54. The method of 53, wherein at least 80% of the cells are multipotent stem cells or progeny thereof and are capable of regenerating skin.
55. The method of 54, wherein at least 90% of the cells are are multipotent stem cells or progeny thereof and are capable of regenerating skin.
56. The method of 55, wherein at least 95% of the cells are multipotent stem cells or progeny thereof and are capable of regenerating skin.
57. The method of 53, wherein said stem cells are substantially purified from hair follicles or dermal papilla-containing portions thereof.
58. The method of 53, wherein said multipotent stem cells express at least one protein selected from the group consisting of nestin, WNT-1, vimentin, fibronectin, S100, slug, snail, twist, Pax3, Sox9, Dermo, and SHOX2.
59. The method of 53, wherein said multipotent stem cells do not express measurable levels of p75NTR.
60. The method of 53, wherein said multipotent stem cells do not express measurable levels of at least one protein selected from the group consisting of tyrosinase, c-kit, tryp1, DCT, MBP, P0, and SOX10.
61. The method of 53, wherein said mammal has a condition characterized by a damaged skin.
62. The method of 61, wherein said condition is the result of accidental injury, surgical trauma, a burn wound, an incisional wound, or a donor site wound from skin transplant.
63. The method of 53, wherein said cells are from said mammal.
64. A method of making hair follicles, said method comprising culturing multipotent stem cells under conditions that induce said stem cells to differentiate into hair follicles.
65. The method of 53, wherein said stem cells are substantially purified from hair follicles or dermal papilla-containing portions thereof.
66. The method of 53, wherein said multipotent stem cells express at least one protein selected from the group consisting of nestin, WNT-1, vimentin, fibronectin, S100, slug, snail, twist, Pax3, Sox9, Dermo, and SHOX2.
67. The method of 53, wherein said multipotent stem cells do not express measurable levels of p75NTR.
68. The method of 53, wherein said multipotent stem cells do not express measurable levels of at least one protein selected from the group consisting of tyrosinase, c-kit, tryp1, DCT, MBP, P0, and SOX10.

## Claims

1. A population of cells for use in a method of regenerating skin in a mammal, wherein at least 30% of said cells are multipotent stem cells that are capable of regenerating skin.

2. The population of claim 1, wherein at least 80% of said cells are multipotent stem cells.

3. The population of claim 2, wherein at least 90% of said cells are multipotent stem cells.

4. The population of claim 3, wherein at least 95% of said cells are multipotent stem cells.

5. The population of claims 1-4, wherein said stem cells are substantially purified from hair follicles or dermal papilla-containing portions thereof.

6. The population of claims 1-5, wherein said multipotent stem cells express at least one protein selected from the group consisting of nestin, WNT-1, vimentin, fibronectin, S100, slug, snail, twist, Pax3, Sox9, Dermo, and SHOX2.

7. The population of claims 1-6, wherein said multipotent stem cells do not express measurable levels of p75NTR.

8. The population of claims 1-7, wherein said multipotent stem cells do not express measurable levels of at least one protein selected from the group consisting of tyrosinase, c-kit, tryp1, DCT, MBP, P0, and SOX10.

9. The population of claims 1-8, wherein said mammal has a condition **characterized by** a damaged skin.

10. The population of claim 9, wherein said condition is the result of accidental injury, surgical trauma, a burn wound, an incisional wound, or a donor site wound from skin transplant.

11. The population of claims 1-10, wherein said cells are from said mammal.

12. The population of claims 1-11, wherein said cells are substantially purified from a hair follicle or a dermal papilla-containing portion thereof by a method comprising the steps of:
(a) providing a hair follicle or a dermal papilla-containing portion thereof from a mammal;
(b) culturing said hair follicle or said portion in conditions under which multipotent stem cells grow and proliferate non-adherently and at least 25% of the cells that are not multipotent stem cells die or adhere to the culture substrate; and
(c) separating non-adherent cells from adherent cells and collecting non-adherent cells; and
(d) continuing culture step (b) and (c) until at least 30% of collected cells are multipotent stem cells or progeny of said multipotent stem cells.

13. The population of claims 1-12, wherein said mammal is a human.


SEQUENCE LISTING

<110> The Hospital For Sick Children

<120> Methods of Making and Using Skin-Derived
  Stem Cells

<130> C64438PCEP

<140> EP 05706434.7
<141> 2005-01-27

<150> 60/539,556
<151> 2004-01-27

<160> 32

<170> FastSEQ for Windows Version 4.0

<210> 1
<211> 24
<212> DNA
<213> Artificial Sequence

<220>
<223> Primer

<400> 1
ggaggcggat ctagaaagga agga                                          24

<210> 2
<211> 22
<212> DNA
<213> Artificial Sequence

<220>
<223> Primer

<400> 2
cccccggaat gagatggttg aa                                            22

<210> 3
<211> 24
<212> DNA
<213> Artificial Sequence

<220>
<223> Primer

<400> 3
cgtcggcagc tccactccac tctc                                          24

<210> 4
<211> 24
<212> DNA
<213> Artificial Sequence

<220>
<223> Primer

<400> 4
tcttcagggc acccaggctc acat                                          24

<210> 5
<211> 23
<212> DNA
<213> Artificial Sequence

<220>
<223> Primer

<400> 5
cgccccgctc ctctgctcta ccc                                              23

<210> 6
<211> 20
<212> DNA
<213> Artificial Sequence

<220>
<223> Primer

<400> 6
gccgccgccg ccaccacctc                                                  20

<210> 7
<211> 18
<212> DNA
<213> Artificial Sequence

<220>
<223> Primer

<400> 7
cggcgccgtc gtccttct                                                    18

<210> 8
<211> 24
<212> DNA
<213> Artificial Sequence

<220>
<223> Primer

<400> 8
ggcctggcac tggtatctct tcac                                             24

<210> 9
<211> 24
<212> DNA
<213> Artificial Sequence

<220>
<223> Primer

<400> 9
ccgcccatca cccgctcgca atac                                             24

<210> 10
<211> 23
<212> DNA
<213> Artificial Sequence

<220>
<223> Primer

<400> 10
gcccctcctc gctgatactg gtg                                              23

<210> 11
<211> 21
<212> DNA
<213> Artificial Sequence

<220>

<223> Primer

<400> 11
gtgcggggtg ggctcaggac t                                                21

<210> 12
<211> 22
<212> DNA
<213> Artificial Sequence

<220>
<223> Primer

<400> 12
ccacaaggcc cacaaccaca gc                                               22

<210> 13
<211> 19
<212> DNA
<213> Artificial Sequence

<220>
<223> Primer

<400> 13
ccgccgccgc caagaccac                                                   19

<210> 14
<211> 22
<212> DNA
<213> Artificial Sequence

<220>
<223> Primer

<400> 14
tccccaaacc cgctcctaca aa                                               22

<210> 15
<211> 22
<212> DNA
<213> Artificial Sequence

<220>
<223> Primer

<400> 15
acccggggga cgtactcatc ac                                               22

<210> 16
<211> 20
<212> DNA
<213> Artificial Sequence

<220>
<223> Primer

<400> 16
cgggaagcgg acagcagaag                                                  20

<210> 17
<211> 18
<212> DNA
<213> Artificial Sequence

<220>
<223> Primer

<400> 17
gccgccaacc gcaaccat                                                    18

<210> 18
<211> 22
<212> DNA
<213> Artificial Sequence

<220>
<223> Primer

<400> 18
gatccggctc tcctcccctt cc                                               22

<210> 19
<211> 18
<212> DNA
<213> Artificial Sequence

<220>
<223> Primer

<400> 19
tggccccggg gacacttc                                                    18

<210> 20
<211> 20
<212> DNA
<213> Artificial Sequence

<220>
<223> Primer

<400> 20
gccgctgacc accccaccat                                                  20

<210> 21
<211> 24
<212> DNA
<213> Artificial Sequence

<220>
<223> Primer

<400> 21
cctggctgcc ctgctcttct cttc                                             24

<210> 22
<211> 22
<212> DNA
<213> Artificial Sequence

<220>
<223> Primer

<400> 22
ccccgatcac tgctcccaac ac                                               22

<210> 23
<211> 21
<212> DNA
<213> Artificial Sequence

<220>
<223> Primer

<400> 23
gcggcgctac agcaagaaat c                                                21

<210> 24
<211> 21
<212> DNA
<213> Artificial Sequence

<220>
<223> Primer

<400> 24
ccatgcgcca cacggagaag g                                                21

<210> 25
<211> 20
<212> DNA
<213> Artificial Sequence

<220>
<223> Primer

<400> 25
ccacgcaaag ccaagacgac                                                  20

<210> 26
<211> 20
<212> DNA
<213> Artificial Sequence

<220>
<223> Primer

<400> 26
gaaaccggcc tgctcatcct                                                  20

<210> 27
<211> 21
<212> DNA
<213> Artificial Sequence

<220>
<223> Primer

<400> 27
tggaaggcac agcagtttac c                                                21

<210> 28
<211> 20
<212> DNA
<213> Artificial Sequence

<220>
<223> Primer

<400> 28
tcatggccca cacgattcac                                                  20

<210> 29
<211> 20
<212> DNA
<213> Artificial Sequence

<220>
<223> Primer

<400> 29
   ccccctcgcc atgaagaagc                                                 20

<210> 30

<211> 19
<212> DNA
<213> Artificial Sequence

<220>
<223> Primer

<400> 30
cagccgcccc acaaccagt                                                    19

<210> 31
<211> 21
<212> DNA
<213> Artificial Sequence

<220>
<223> Primer

<400> 31
gtcttcacca ccatggagaa g                                                 21

<210> 32
<211> 21
<212> DNA
<213> Artificial Sequence

<220>
<223> Primer

<400> 32
gtgatggcat ggactgtggt c                                                 21